# EUROPEAN PATENT SPECIFICATION

(11) **EP 3 398 958 B1**
(45) Date of publication and mention of the grant of the patent: **23.02.2022**
(21) Application number: 16881284.0
(22) Date of filing: 30.12.2016
(51) Int. Cl.: C07K 14/56, C07K 14/71, C07K 14/435, A61K 47/64, A61P 5/00, A61P 37/00, A61P 37/04

(54) **POLYAMINOACID, PROTEIN-POLYAMINOACID CONJUGATE AND PREPARATION METHOD THEREFOR**
POLYAMINOSÄURE, PROTEIN-POLYAMINOSÄURE-KONJUGAT UND HERSTELLUNGSVERFAHREN DAFÜR
POLYAMINO-ACIDE, CONJUGUÉ PROTÉINE-POLYAMINO-ACIDE ET PROCÉDÉ DE PRÉPARATION CORRESPONDANT

(30) Priority: 30.12.2015 CN 201511020841; 05.07.2016 CN 201610523202
(43) Date of publication of application: 07.11.2018
(73) Proprietor: Peking University, Beijing 100871 (CN)
(72) Inventor: LU, Hua, Haidian District Beijing 100871 (CN); YUAN, Jingsong, Haidian District Beijing 100871 (CN); HOU, Yingqin, Haidian District Beijing 100871 (CN); ZHOU, Yu, Haidian District Beijing 100871 (CN)
(74) Representative: Gill Jennings & Every LLP
(86) International application number: PCT/CN2016/113590
(87) International publication number: WO 2017/114495

(56) References cited:
- EP-A1- 2 784 080
- WO-A1-00/42175
- WO-A2-99/28343
- WO-A2-2009/133545
- CN-A- 1 706 865
- CN-A- 101 002 946
- US-A1- 2006 233 747
- US-B1- 6 849 428
- EVANS T C ET AL: "SEMISYNTHESIS OF CYTOTOXIC PROTEINS USING A MODIFIED PROTEIN SPLICING ELEMENT", PROTEIN SCIENCE, WILEY, US, vol. 7, 1 January 1998 (1998-01-01), pages 2256-2264, XP002944418, ISSN: 0961-8368
- CATHERINE NAVARRE ET AL: "Expression and Secretion of Recombinant Outer-surface Protein A from the Lyme Disease Agent, Borrelia burgdorferi, in Nicotiana tabacum Suspension Cells", TRANSGENIC RESEARCH, KLUWER ACADEMIC PUBLISHERS-PLENUM PUBLISHERS, NE, vol. 15, no. 3, 1 June 2006 (2006-06-01), pages 325-335, XP019409550, ISSN: 1573-9368, DOI: 10.1007/S11248-006-0002-7
- PAUL W. R. HARRIS ET AL: "Chemical synthesis of a polypeptide backbone derived from the primary sequence of the cancer protein NY-ESO-1 enabled by kinetically controlled ligation and pseudoprolines : Chemical Synthesis of a Polypeptide Backbone", BIOPOLYMERS, vol. 104, no. 2, 1 March 2015 (2015-03-01) , pages 116-127, XP055597194, US ISSN: 0006-3525, DOI: 10.1002/bip.22621
- TOLBERT T J ET AL: "A new strategy for glycoprotein synthesis: ligation of synthetic glycopeptides with truncated proteins expressed in E. coli as TEV protease cleavable fusion protein", BIOORGANIC & MEDICINAL CHEMISTRY, PERGAMON, GB, vol. 13, no. 3, 1 February 2005 (2005-02-01), pages 909-915, XP027637793, ISSN: 0968-0896 [retrieved on 2005-02-01]
- DATABASE COMPENDEX [Online] ENGINEERING INFORMATION, INC., NEW YORK, NY, US; September 2004 (2004-09), NISHIDE K ET AL: "Development of odorless sulfur reagents and their applications", XP002792202, Database accession no. E2004508711332 & YUKI GOSEI KAGAKU KYOKAISHI/JOURNAL OF SYNTHETIC ORGANIC CHEMISTRY SEPTEMBER 2004 SOCIETY OF SYNTHETIC ORGANIC CHEMISTRY JP, vol. 62, no. 9, September 2004 (2004-09), pages 895-910, DOI: 10.5059/YUKIGOSEIKYOKAISHI.62.895
- MUIR T W ET AL: "Expressed protein ligation: A general method for protein engineering", PROCEEDINGS OF THE NATIONAL ACADEMY OF SCIENCES OF THE UNITED STATES OF AMERICA, NATIONAL ACADEMY OF SCIENCES, US, vol. 95, no. 12, 9 June 1998 (1998-06-09), pages 6705-6710, XP002257746, ISSN: 0027-8424, DOI: 10.1073/PNAS.95.12.6705
- ZHAO ET AL: "Circumventing the heterogeneity and instability of human serum albumin-interferon-@a2b fusion protein by altering its orientation", JOURNAL OF BIOTECHNOLOGY, ELSEVIER, AMSTERDAM, NL, vol. 131, no. 3, 6 September 2007 (2007-09-06), pages 245-252, XP022232027, ISSN: 0168-1656, DOI: 10.1016/J.JBIOTEC.2007.04.016
- XINYING JIA ET AL: "Semienzymatic Cyclization of Disulfide-rich Peptides Using Sortase A", JOURNAL OF BIOLOGICAL CHEMISTRY, vol. 289, no. 10, 14 January 2014 (2014-01-14), pages 6627-6638, XP055597299, US ISSN: 0021-9258, DOI: 10.1074/jbc.M113.539262
- YANXIN J. WANG ET AL: "Chemoselective modifications for the traceless ligation of thioamide-containing peptides and proteins", ORGANIC & BIOMOLECULAR CHEMISTRY, vol. 14, no. 26, 1 January 2016 (2016-01-01), pages 6262-6269, XP055596958, ISSN: 1477-0520, DOI: 10.1039/C6OB01020B
- CHEN, PENG: 'A Concise Synthesis of Site-Specific and Topologically-Defined Protein-Poly (Amino Acid) Conjugates' ACTAPOLYMERICASINICA 30 November 2016, ISSN 1000-3304 pages 1465 - 1467, XP055508478
- WANG, MINGZHI ET AL.: 'Progress in Syntheses and Applications of Polypeptides and Polypeptide-Based Copolymers by NCA Polymerization' ACTAPOLYMERICASINICA 30 September 2014, ISSN 1000-3304 pages 1183 - 1194, XP055396302
- YANG, S.R. ET AL.: 'Histidine-Conjugated Poly (Amino Acid) Derivatives for the Novel Endosomolytic Delivery Carrier of Doxorubicin' JOURNAL OF CONTROLLED RELEASE vol. 114, no. 1, 10 August 2006, ISSN 0168-3659 pages 60 - 68, XP024957573
- Duy P. Nguyen ET AL: "Genetically Encoded 1,2-Aminothiols Facilitate Rapid and Site-Specific Protein Labeling via a Bio-orthogonal Cyanobenzothiazole Condensation", JOURNAL OF THE AMERICAN CHEMICAL SOCIETY, vol. 133, no. 30, 3 August 2011 (2011-08-03), pages 11418-11421, XP055713203, US ISSN: 0002-7863, DOI: 10.1021/ja203111c
- Yingqin Hou ET AL: "A Concise Approach to Site-Specific Topological Protein-Poly(amino acid) Conjugates Enabled by in Situ -Generated Functionalities", JOURNAL OF THE AMERICAN CHEMICAL SOCIETY, vol. 138, no. 34, 18 August 2016 (2016-08-18), pages 10995-11000, XP055508478, US ISSN: 0002-7863, DOI: 10.1021/jacs.6b05413
- DATABASE COMPENDEX [Online] ENGINEERING INFORMATION, INC., NEW YORK, NY, US; September 2004 (2004-09), NISHIDE K ET AL: "Development of odorless sulfur reagents and their applications", Database accession no. E2004508711332 & NISHIDE K ET AL: "Development of odorless sulfur reagents and their applications", YUKI GOSEI KAGAKU KYOKAISHI/JOURNAL OF SYNTHETIC ORGANIC CHEMISTRY SEPTEMBER 2004 SOCIETY OF SYNTHETIC ORGANIC CHEMISTRY JP, vol. 62, no. 9, September 2004 (2004-09), pages 895-910, DOI: 10.5059/YUKIGOSEIKYOKAISHI.62.895

## Description

### TECHNICAL FIELD

The disclosure relates to the biopharmaceutical field, specifically to a site-specific protein-poly(amino acid) conjugate, a poly(amino acid) for preparing such conjugate, and the preparation process thereof.

### BACKGROUND

Protein (such as antibodies, interferons) have been widely used in the biopharmaceutical field, such as targeted therapy, clinical diagnosis *etc.* Compared with other drugs, proteins have better specificity, quick effect and low toxicity. However, the efficacy and application of proteins have been limited to a certain extent due to their poor stability, short blood circulation time and other defects.

At present, the system which is often researched for improving protein stability and prolonging its blood circulation time is a protein-polymer conjugate. Proteins tend to have relatively short blood circulation time due to protease mediated degradation in vivo. Therefore, a water-soluble polymer material grafted on the surface of a protein will provide certain protecting effect on the protein, which not only reduces the immunogenicity of the protein, but also reduces the protease mediated degradation of proteins to prolong the blood circulation time. For example, interferon-α2a is a drug for treating chronic hepatitis C, and grafting one 40 kDa polyethylene glycol chain on its surface will significantly prolong the blood circulation time. An interferon-α2a-polyethylene glycol conjugate has been used clinically.

However, at present, polyethylene glycol (PEG) is a polymer fragment commonly used in protein-polymer conjugates that have been applied clinically. PEG has advantages such as low immunogenicity, good water solubility, *etc.* However, with the wide application of PEG in cosmetic, food, pharmaceutical field or the like, anti-PEG antibodies have been produced in bodies of most people, which will in turn accelerate the clearance of PEG modified proteins in blood. What is more serious is that PEG is not degradable in human body, and long-term administration will lead to cumulative toxicity. Therefore, it is an urgent need of finding a polymer material which is biodegradable and has good biocompatibility.

Poly(amino acid) (PAA) is a sort of polymeric material widely considered promising for protein modification due to its good biocompatibility, main chain degradability, and facile side chain modification. More importantly, PAA has various advantages such as main chain structure of polypeptide, good biocompatibility, low immunogenicity and the like, and thus is very suitable for the modification of proteins.

Therefore, the disclosure provides a protein-poly(amino acid) conjugate and a protein-poly(amino acid) cyclic conjugate to overcome the defect existing in the field.

Evans et al., 1998 (Protein Science, pages 2256-2264), relates to the semisynthesis of cytotoxic proteins using a modified protein splicing element. Navarre et al., 2006 (Transgenic Research, pages 325-335), relates to the expression and secretion of recombinant outer-surface Protein A from the Lyme Disease Agent, Borrelia burgdorferi, in Nicotiana tabcum suspension cells. WO 99/28343 A2 relates to hydrophobically modified protein compositions and methods of making said compositions. WO 00/42175 A1 relates to methods for making proteins containing free cysteine residues. Harris et al., 2015 (Biopolymers, pages 116-127) relates to the chemical synthesis of a polypeptide backbone derived from the primary sequence of the cancer protein NY-ESO-1 enabled by kinetically controlled ligation and pseudoprolines. Tolbert et al., 2005 (Bioorganic and Medicinal Chemistry, pages 909-915) relates to a new strategy for glycoprotein synthesis involving the ligation of synthetic glycopeptides with truncated proteins expressed in E.coli as TEV protease cleavable fusion protein. Nishide et al., 2004 (Journal of Synthetic Organic Chemistry, pages 895-910) relates to the development of odorless sulfur reagents and their applications. Muir et al., 1998 (National Academy of Sciences, pages 6705-6710) relate to a general method for protein engineering. US 6 849 428 B1 relates to intein-mediated protein ligation of expressed proteins. Zheo et al., 2007 (Journal of Biotechnology, pages 245-252) relates to circumventing the heterogeneity and instability of human serum albumin-interferon-a2b protein by altering its orientation. EP 2 784 080 A1 relates to peptide-hinge-free flexible antibody-like molecules. US 2006/233747 A1 relates to polymer-modified synthetic proteins. Xinying et al., 2014 (Journal of Biological Chemistry, pages 6627-6638) relates to the semienzymatic cyclization of disulfide-rich peptides using sortase A. Yingqin et al., 2016 (Journal of the American Chemical Society, pages 10995-11000) relates to a concise approach to site-specific topological protein-poly(amino acid) conjugates enabled by in situ-generated functionalities. Nguyen et al., 2011 (Journal of the American Chemical Society, pages 11418-11421) relates to genetically encoded 1, 2-aminmothiols facilitating rapid and site-specific protein labelling via a bio-orthogonal cyanobenzothiazole condensation.

### SUMMARY

In an aspect, the disclosure provides a protein-poly(amino acid) conjugate, having a structure represented by general formula 1: 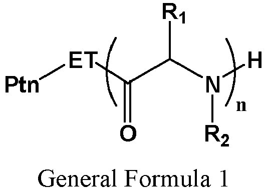 wherein
Ptn represents a protein;
ET is 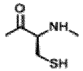 when it is connected with the N-terminus of the Ptn, and is 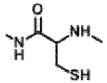 when it is connected with a site other than the N-terminus of the Ptn;
R₁ independently represents, at each occurrence, a side chain of a non-natural amino acid;
R₂ independently represents, at each occurrence, hydrogen or C₁-C₁₀ alkyl, C₂-C₁₀ alkenyl, C₂-C₁₀ alkynyl; and
n is an integer selected from 1 to 200.

In an embodiment, a protein-poly(amino acid) conjugate described herein has a structure represented by general formula 2: 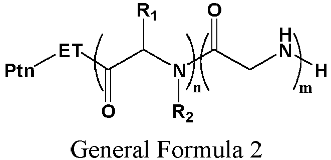 wherein
Ptn, ET, R₁ and R₂ are as defined herein;
n is an integer selected from 1 to 200; and
m is an integer selected from 1 to 30.

In another aspect, the disclosure provides a protein-poly(amino acid) cyclic conjugate, having a structure represented by general formula 3: 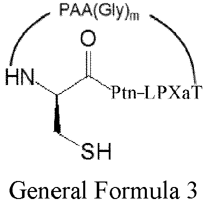 wherein
Ptn represents a protein having a cysteine residue at the N-terminus and an LPXaT sequence at the C-terminus, in which Xa represents one or more amino acids; PAA(Gly)ₘ is a structure represented by general formula 4: 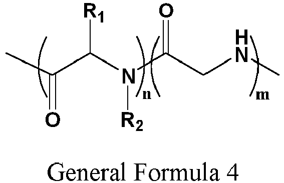 wherein
R₁ independently represents, at each occurrence, r a side chain of a non-natural amino acid;
R₂ independently represents, at each occurrence, hydrogen or C₁-C₁₀ alkyl, C₂-C₁₀ alkenyl, C₂-C₁₀ alkynyl;
n is an integer selected from 10 to 200; and
m is an integer selected from 1 to 30.

In yet another aspect, the disclosure provides a poly(amino acid) compound for preparing the protein-poly(amino acid) (cyclic) conjugate, and the compound has a structure represented by general formula 5: 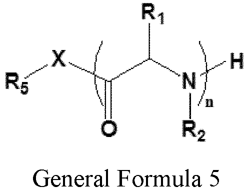 wherein
X represents sulphur or selenium;
R₁ independently represents, at each occurrence, a side chain of a non-natural amino acid;
R₂ independently represents, at each occurrence, hydrogen or C₁-C₁₀ alkyl, C₂-C₁₀ alkenyl, C₂-C₁₀ alkynyl;
R₅ represents substituted or unsubstituted C₁-C₃₀ alkyl, substituted or unsubstituted C₂-C₃₀ alkenyl, substituted or unsubstituted C₂-C₃₀ alkynyl, substituted or unsubstituted C₃-C₃₀ cycloalkyl, substituted or unsubstituted C₃-C₃₀ cycloalkenyl, substituted or unsubstituted C₁-C₃₀ heteroalkyl, substituted or unsubstituted C₂-C₃₀ heteroalkenyl, substituted or unsubstituted C₂-C₃₀ heteroalkynyl, substituted or unsubstituted C₁-C₃₀ heterocycloalkyl, substituted or unsubstituted C₂-C₃₀ heterocycloalkenyl, substituted or unsubstituted C₆-C₃₀ aryl, or substituted or unsubstituted C₅-C₃₀ heteroaryl; and
n is an integer selected from 1 to 200.

According an embodiment of the disclosure, the poly(amino acid) compound as described herein has a structure represented by general formula 6 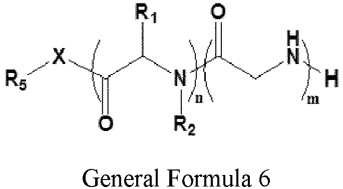 wherein
X, R₁, R₂ and R₅ are as defined herein;
n is an integer selected from 10 to 200; and
m is an integer selected from 1 to 30.

In another aspect, the disclosure provides a method for preparing the protein-poly(amino acid) conjugate of General Formula 1 and 2, comprising: (1) initiating polymerization of a N-carboxyanhydride by an initiator R₅XY to obtain a poly(amino acid) having a carbon structure; and (2) mixing the poly(amino acid) with a protein containing a 1,2-mercaptoethylamine structure to obtain a protein-poly(amino acid) conjugate through native chemical ligation, wherein X represents sulphur or selenium; Y represents hydrogen or trialkylsilyl, wherein the alkyl moiety in the trialkylsilyl is C₁-C₁₀ alkyl; and R₅ represents substituted or unsubstituted C₁-C₃₀ alkyl, substituted or unsubstituted C₂-C₃₀ alkenyl, substituted or unsubstituted C₂-C₃₀ alkynyl, substituted or unsubstituted C₃-C₃₀ cycloalkyl, substituted or unsubstituted C₃-C₃₀ cycloalkenyl, substituted or unsubstituted C₁-C₃₀ heteroalkyl, substituted or unsubstituted C₂-C₃₀ heteroalkenyl, substituted or unsubstituted C₂-C₃₀ heteroalkynyl, substituted or unsubstituted C₁-C₃₀ heterocycloalkyl, substituted or unsubstituted C₂-C₃₀ heterocycloalkenyl, substituted or unsubstituted C₆-C₃₀ aryl, or substituted or unsubstituted C₅-C₃₀ heteroaryl.

An embodiment of the disclosure provides a method for preparing the protein-poly(amino acid) cyclic conjugate of General Formula 3 and 4, comprising: (1) initiating polymerization of a N-carboxyanhydride and a glycine N-carboxyanhydride by an initiator R₅XY to obtain a block poly(amino acid) having a phenylthioester structure at the C-terminus and a block polyglycine structure at the N-terminus; and (2) mixing the block poly(amino acid) with a protein having a cysteine residue at the N-terminus and a LPXaTG sequence at the C-terminus to successively conduct native chemical ligation and sortase mediated ligation to achieve cyclization of the protein, wherein the LPXaTG sequence is a recognition site of the sortase, wherein X represents sulphur; Y represents hydrogen or trialkylsilyl, wherein the alkyl moiety in the trialkylsilyl is C₁-C₁₀ alkyl; and R₅ represents phenyl group .

In other aspects, the disclosure provides the use of the protein-poly(amino acid) (cyclic) conjugate as described herein for preparing a protein.

### BRIEF DESCRIPTION OF THE DRAWINGS

Embodiments illustrated herein are further described in the following description in conjunction with the accompanying drawings. However, the accompanying drawings are only provided to enable those skilled in the art to better understand the disclosure, rather than limit the scope of the disclosure.
Fig. 1 is a schematic diagram of a site-specific protein-poly(amino acid) conjugate according to an embodiment of the disclosure;
Fig. 2 is a schematic diagram of a cyclization reaction of a protein-poly(amino acid) according to an embodiment of the disclosure;
Fig. 3 is MALDI-TOF plot of poly(amino acid) P1 according to Example 1;
Fig. 4 is MALDI-TOF plot of cyclic conjugate according to Example 15, wherein O represents L-glutamate (ethylene glycol)₃ segment, G represents glycine segment, and the corner mark represents polymerization degree of corresponding segment;
Fig. 5 is UPLC-MS plot of ENLYFQ-Cys-eGFP protein according to Example 16;
Fig. 6 is UPLC-MS plot of Cys-eGFP protein according to Example 18;
Fig. 7 is an electrophoretic diagram of poly(amino acid) P1 (n=7)-eGFP conjugate according to Example 19, showing SDS-PAGE (A) and native PAGE (B);
Fig. 8 is an electrophoretic diagram of an ENLYFQC-eGFP-polyethylene glycol-polyglycine conjugate according to Example 20, showing SDS-PAGE (A) and native PAGE (B);
Fig. 9 is an electrophoretic diagram of a knot-like conjugate of poly(amino acid) P1 (n=7)-eGFP-polyglycine-polyethylene glycol according to Example 21, showing SDS-PAGE (A) and native PAGE (B);
Fig. 10 is an electrophoretic diagram of a dumbbell-like conjugate of eGFP-poly(amino acid) P2 (n=7, m=3)-interferon α according to Example 22, showing SDS-PAGE (A) and native PAGE (B);
Fig. 11 shows characterization of a cyclic conjugate of eGFP-poly(amino acid) P2 (n=7, m=3) according to Example 23: MALDI-TOF (A), SDS-PAGE (B) and western blot (C);
Fig. 12 shows characterization of the enzymatic stability of a Cys-eGFP (A), a poly(amino acid) P2 (n=7, m=3)-eGFP (B) and a cyclic conjugate of eGFP-poly(amino acid) P2 (n=7, m=3) (C) according to Example 24: the blocks in panels A, B and C are proteins or protein-poly(amino acid) conjugate materials;
Fig. 13 is UPLC-MS spectrum of an interferon according to Example 26;
Fig. 14 is an electrophoretic diagram of poly(amino acid) conjugates according to Examples 27 and 28;
Fig. 15 shows characterization of a cyclic conjugate of poly(amino acid) P2 (n=7 or 20, m=3)-interferon α-LPETGLEH₆ according to Example 29: A. SDS-PAGE of the cyclic conjugate of poly(amino acid) P2 (n=7, m=3)-interferon α-LPETGLEH₆; B.
western blot of the cyclic conjugate of poly(amino acid) P2 (n=7, m=3)-interferon α-LPETGLEH₆; C. SDS-PAGE of the cyclic conjugate of poly(amino acid) P2 (n=20,
m=3)-interferon α-LPETGLEH₆; D. western blot of the cyclic conjugate of poly(amino acid) P2 (n=20, m=3)-interferon α-LPETGLEH₆;
Fig. 16 is a column diagram showing Tm value of each protein sample according to Example 30;
Fig. 17 is a graph showing the result of trypsin resistance test according to Example 31;
Fig. 18 is circular dichroism spectrum of a wt-IFN α, a poly(amino acid) P1 (n=100, L-type)-IFNα conjugate and a poly(amino acid) P1 (n=100, D,L-type)-IFNα conjugate according to Example 32;
Fig. 19 is a graph showing plasma concentration of a poly(amino acid)-interferon conjugate according to Example 34;
Figs. 20A and 20B are graphs showing *in vivo* antitumor activities of an interferon-poly(amino acid)-conjugate and a macrocyclic interfereon-poly(amino acid)-cyclic conjugate according to Example 35; and
Fig. 21 is an electrophoretic diagram of a poly(amino acid)-antibody conjugate according to Example 37.

### DETAILED DESCRIPTION OF EMBODIMENTS

The disclosure will be further illustrated by the following specific embodiments. However, the specific embodiments are listed for illustrative purposes only, and not intended to limit the disclosure.

### DEFINITIONS

Hereinafter, unless otherwise defined, all terms (including technical and scientific terms) used herein have the same meanings as commonly understood by one of ordinary skill in the art to which this disclosure belongs. Terms, such as those defined in commonly used dictionaries, should be interpreted as having meanings that are consistent with their meanings in the context of the relevant art, and will not be interpreted in an idealized or overly formal sense unless expressly so defined herein.

As used herein, the term "C₁₋₃₀" means that a main chain of a group has any integral number of carbon atoms within the range of 1 to 30, e.g., 1, 2, 3, 4, 5, 6, 7, 8, 9, 10, 12, 15, 18, 20 or 30 carbon atoms.

As used herein, the term "alkyl" refers to a saturated aliphatic hydrocarbyl group having a straight or branched chain, including without limitation methyl, ethyl, propyl, n-butyl, tert-butyl, pentyl, hexyl, heptyl, octyl, nonyl, decyl, and the like. As used herein, the term "alkenyl" refers to a hydrocarbyl group having at least one carbon-carbon double-bond at one or more sites along the carbon chain of an alkyl group, including without limitation ethenyl, propenyl, butenyl, and the like. As used herein, the term "alkynyl" refers to a hydrocarbyl group having at least one carbon-carbon triple-bond at one or more sites along the carbon chain of an alkyl group, including without limitation ethynyl, propynyl, and the like.

As used herein, the terms "heteroalkyl", "heteroalkenyl" and "heteroalkynyl" separately refer to an alkyl, an alkenyl and an alkynyl containing at least one heteroatom selected from the group consisting of N, O, Si, P and S.

As used herein, the term "cycloalkyl" refers to a monocyclic saturated hydrocarbyl group, including without limitation cyclopropyl, cyclobutyl, cyclopentyl, cyclohexyl and cycloheptyl. As used herein, the term "heterocycloalkyl" refers to a monocarbocyclic group containing at least one heteroatom selected from the group consisting of N, O, Si, P and S as ring-forming atom, including without limitation tetrahydrofuranyl and tetrahydrothienyl.

As used herein, the term "cycloalkenyl" refers to a non-aromatic monocyclic group having carbon atoms and at least one double-bond in its ring, including without limitation cyclopentenyl, cyclohexenyl and cycloheptenyl. As used herein, the term "heterocycloalkenyl" refers to a monocarbocyclic group containing at least one heteroatom selected from the group consisting of N, O, Si, P and S as ring-forming atoms, and at least one double-bond in its ring, including without limitation 4,5-dihydro-1,2,3,4-oxatriazolyl, 2,3-dihydrofuranyl and 2,3-dihydrothienyl.

As used herein, the term "aryl" refers to a group containing a carbocyclic aromatic system, including without limitation phenyl, naphthyl, anthryl, phenanthryl, pyrenyl, and the like. When an aryl includes a plurality of rings, the respective rings may be fused with one another.

As used herein, the term "heteroaryl" refers to a group having a carbocyclic aromatic system containing at least one heteroatom selected from the group consisting of N, O, Si, P and S as ring-forming atom, including without limitation pyridinyl, pyrimidinyl, pyrazinyl, pyridazinyl, triazinyl, quinolyl, isoquinolyl, and the like. When a heteroaryl includes a plurality of rings, the respective rings may be fused with one another.

### PROTEIN-POLY(AMINO ACID) CONJUGATE

An embodiment of the disclosure provides a protein-poly(amino acid) conjugate, having a structure represented by general formula 1: 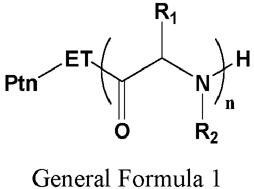 wherein
Ptn represents a protein;
ET is 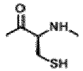 when it is connected with the N-terminus of the Ptn, and is 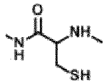 when it is connected with a site other than the N-terminus of the Ptn;
R₁ independently represents, at each occurrence, a side chain of a non-natural amino acid;
R₂ independently represents, at each occurrence, hydrogen or C₁-C₁₀ alkyl, C₂-C₁₀ alkenyl, C₂-C₁₀ alkynyl; and n is an integer selected from 1 to 200.

According to an embodiment of the disclosure, Ptn is a protein selected from the group consisting of an enzyme, a hormone, a cytokine, a monoclonal antibody and/or a protein vaccine. According to another embodiment of the disclosure, the Ptn is a protein, such as a polypeptide hormone, a monoclonal antibody, an interferon, an interleukin, a colony stimulating factor and a recombinant vaccine. According to a further embodiment of the disclosure, the Ptn is interferon α2b. A protein that is suitable for use in the disclosure is not particularly limited, as long as it can be connected with the ET linking group. In theory, any modified protein may be used herein.

According to an embodiment of the disclosure, an enzyme that can be used in the disclosure includes, but is not limited to: a proteolytic enzyme, an amylase, a lipase, a cellulase, a trypsin, a chymotrypsin, a streptokinase, an urokinase, a fibrinolysin, a thrombin, a glutaminase, an arginase, a serine dehydrase, a phenylanlanine ammonialyase, a leucine dehydrogenase, a penicillinase, a superoxide dismutase, a glucanase and/or a dextranase.

According to an embodiment of the disclosure, a hormone that can be used in the disclosure includes, but is not limited to: a hypothalamic hormone, a pituitary hormone, a gastrointestinal hormone, insulin and calcitonin.

According to an embodiment of the disclosure, a cytokine that can be used in the disclosure includes, but is not limited to: an interleukin, an interferon, a colony stimulating factor, a chemokine and/or a growth factor.

According to an embodiment of the disclosure, an interleukin that can be used in the disclosure includes, but is not limited to: IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31 and/or IL-32.

According to an embodiment of the disclosure, an interferon that can be used in the disclosure includes, but is not limited to, an IFN-α, an IFN-β, an IFN-γα, an IFN-λ and subtypes thereof.

According to an embodiment of the disclosure, a colony stimulating factor that can be used in the disclosure includes, but is not limited to: a granulocyte colony stimulating factor, a macrophage colony stimulating factor, a granulocyte-macrophage colony stimulating factor, a pleuripotent colony stimulating factor, a stem cell factor, a leukemia inhibitory factor and/or an erythrogenin.

According to an embodiment of the disclosure, a growth factor that can be used in the disclosure includes, but is not limited to: an epidermal growth factor, a transforming growth factor, an insulin-like growth factor and/or a nerve growth factor.

According to an embodiment of the disclosure, a monoclonal antibody that can be used in the disclosure includes, but is not limited to: trastuzumab, cetuximab, daclizumab, tanezumab, abagovomab, adecatumumab, afutuzumab, alemtuzumab, alacizumab, amatuximab, apolizumab, bavituximab, bectumomab, belimumab, bevacizumab, bivatuzumab mertansine, brentuximab vedotin, cantuzumab mertansin, cantuzumab ravtansine, capromab pendetide, catumaxomab, citatuzumab bogatox, cixutumumab, conatumumab, dacetuzumab, dalotuzumab, detumomab, ecromeximab, edrecolomab, elotuzumab, ensituximab, epratuzumab, ertumaxomab, etaracizumab, farletuzumab, figitumumab, galiximab, gemtuzumab, girentuximab, glembatumumab vedotin, ibritumomab, igovomab, indatuximab ravtansine, intetumumab, inotuzumab ozogamicin, ipilimumab, iratumumab, labetuzumab, lexatumumab, lintuzumab, lorvotuzumab mertansine, lucatumumab, lumiliximab, mapatumumab, matuzumab, milatuzumab, mitumomab, mogamulizumab, nacolomab tafenatox, naptumomab estafenatox, necitumumab, nimotuzumab, nivolumab, ofatumumab, omalizumab, oportuzumab monatox, oregovomab, pemtumomab, patritumab, pertuzumab, pritumumab, racotumomab, ramucirumab, rilotumumab, rituximab, robatumumab, omalizumab, sibrotuzumab, siltuximab, taplitumomab paptox, tenatumomab, teprotumumab, ticilimumab, tremelimumab, tigatuzumab, tositumomab, tucotuzumab celmoleukin, urelumab, veltuzumab, volociximab, votumumab and zalutumumab, including antigen binding fragments thereof.

According to an embodiment of the disclosure, a protein vaccine that can be used in the disclosure includes, but is not limited to: diphtheria toxoid, tetanus toxoid, anthrax secreted protein vaccine and/or plasma derived hepatitis B vaccine.

According to an embodiment of the disclosure, R₁ represents, at each occurrence, a side chain of a non-natural amino acid, where the corresponding natural amino acid is selected from the group consisting of glycine, alanine, valine, leucine, isoleucine, proline, phenylalanine, tyrosine, tryptophan, serine, threonine, cysteine, methionine, aspartic acid, glutamic acid, lysine, arginine and histidine. According to this embodiment of the disclosure, R₁ represents, at each occurrence, a side chain of a non-natural amino acid, such as those obtained from artificially modifying the above natural amino acid. E.g. R₁ represents, at each occurrence, a side chain of tyrosine, serine, threonine, cysteine, aspartic acid and/or glutamic acid that has been modified with oligo(ethylene glycol) (polymerization degree: 2-10, such as triethylene glycol, OEG₃), phosphate, propenyloxybenzyl ester or allyl triethylene glycol. According to other embodiment of the disclosure, R₁ represents, at each occurrence, a side chain of triethylene glycol (OEG₃) modified tyrosine, serine, threonine, cysteine, aspartic acid and/or glutamic acid. The natural amino may be modified and then used for the conjugate in the disclosure to improve stability.

According to an embodiment of the disclosure, R₂ represents, at each occurrence, hydrogen or C₁-C₁₀ alkyl. According to another embodiment of the disclosure, R₂ represents, at each occurrence, hydrogen, methyl, ethyl, n-propyl, isopropyl, n-butyl, tert-butyl, pentyl or hexyl. According to yet another embodiment of the disclosure, R₂ represents hydrogen or methyl.

According to an embodiment of the disclosure, n represents an integer selected from 1 to 200. According to another embodiment of the disclosure, n represents an integer selected from 1 to 150. According to yet another embodiment of the disclosure, n represents an integer selected from 1 to 100. According to other embodiment of the disclosure, n represents an integer selected from 1 to 50. In theory, n may be any numerical value as long as the resulting conjugate stably exists and falls within the scope of the claims, and can be specifically set by those skilled in the art based on practical application. Moreover, such choice should fall within the scope of abilities of those skilled in the art.

An embodiment of the disclosure provides a protein-poly(amino acid) conjugate, having a structure represented by general formula 2: 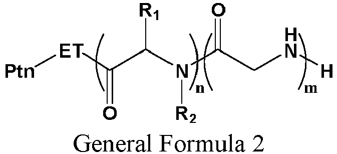 wherein
Ptn, ET, R₁ and R₂ are as defined in general formula 1;
n is an integer selected from 1 to 200; and
m is an integer selected from 1 to 30.

According to an embodiment of the disclosure, n represents an integer selected from 1 to 150. According to another embodiment of the disclosure, n represents an integer selected from 1 to 100. According to yet another embodiment of the disclosure, n represents an integer selected from 1 to 50. According to an embodiment of the disclosure, m represents an integer selected from 1 to 20. According to another embodiment of the disclosure, m represents an integer selected from 1 to 10. As mentioned above, n and m may be any numerical value, and are not limited to the specific scopes enumerated herein, provided that the resulting conjugate may stably exist and falls within the scope of the claims. n and m can be specifically set by those skilled in the art based on practical application. Moreover, such choice should fall within the scope of abilities of those skilled in the art.

### PROTEIN-POLY(AMINO ACID) CYCLIC CONJUGATE

An embodiment of the disclosure provides a protein-poly(amino acid) cyclic conjugate, having a structure represented by general formula 3:

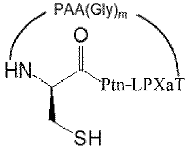

### General Formula 3 wherein

Ptn represents a protein, having a cysteine residue at the N-terminus and an LPXaT sequence at the C-terminus, where Xa represents one or more amino acids; PAA(Gly)ₘ is a structure represented by general formula 4:

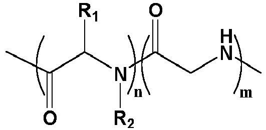

### General Formula 4wherein

R₁ independently represents, at each occurrence, a side chain of a non-natural amino acid;
R₂ independently represents, at each occurrence, hydrogen or C₁-C₁₀ alkyl, C₂-C₁₀ alkenyl, C₂-C₁₀ alkynyl;
n is an integer selected from 10 to 200; and
m is an integer selected from 1 to 30.

According to an embodiment of the disclosure, Ptn is a protein as described above, where the protein has a cysteine residue at the N-terminus and an LPXaT sequence at the C-terminus, or may be modified to have a cysteine residue at the N-terminus and an LPXaT sequence at the C-terminus. According to another embodiment of the disclosure, the Ptn is an interferon α2b having a cysteine residue at the N-terminus and an LPXaT sequence at the C-terminus. According to yet another embodiment of the disclosure, the Ptn is a protein having an amino acid sequence of SEQ ID No. 1. According to other embodiment of the disclosure, the Ptn is as defined in general formula 1.

According to an embodiment of the disclosure, R₁ represents, at each occurrence, a side chain of a non-natural amino acid, where the corresponding natural amino acid is selected from the group consisting of glycine, alanine, valine, leucine, isoleucine, proline, phenylalanine, tyrosine, tryptophan, serine, threonine, cysteine, methionine, aspartic acid, glutamic acid, lysine, arginine and histidine. According to this embodiment of the disclosure, R₁ represents, at each occurrence, a side chain of a non-natural amino acid, such as those obtained from artificially modifying the above natural amino acid. According to yet another embodiment of the disclosure, R₁ represents, at each occurrence, a side chain of tyrosine, serine, threonine, cysteine, aspartic acid and/or glutamic acid that has been modified with oligo(ethylene glycol) (polymerization degree: 2-10, such as triethylene glycol, OEG₃), phosphate, propenyloxybenzyl ester or allyl triethylene glycol. According to other embodiment of the disclosure, R₁ represents, at each occurrence, a side chain of triethylene glycol (OEG₃) modified tyrosine, serine, threonine, cysteine, aspartic acid and/or glutamic acid.

According to an embodiment of the disclosure, Xa may be one or more selected from the group consisting of glycine, alanine, valine, leucine, isoleucine, proline, phenylalanine, tyrosine, tryptophan, serine, threonine, cysteine, methionine, aspartic acid, glutamic acid, lysine, arginine and histidine.

According to an embodiment of the disclosure, R₂ represents, at each occurrence, hydrogen or C₁-C₁₀ alkyl. According to another embodiment of the disclosure, R₂ represents, at each occurrence, hydrogen, methyl, ethyl, n-propyl, isopropyl, n-butyl, tert-butyl, pentyl, neopentyl or hexyl. According to yet another embodiment of the disclosure, R₂ represents hydrogen.

According to an embodiment of the disclosure, n represents an integer selected from 10 to 200. According to another embodiment of the disclosure, n represents an integer selected from 10 to 150. According to yet another embodiment of the disclosure, n represents an integer selected from 10 to 100. According to other embodiment of the disclosure, n represents an integer selected from 10 to 50. According to an embodiment of the disclosure, m represents an integer selected from 1 to 20. According to another embodiment of the disclosure, m represents an integer selected from 1 to 10. As mentioned above, n and m may be any numerical value, provided that the resulting conjugate may stably exist and falls within the scope of the claims. n and m can be specifically set by those skilled in the art based on practical application. Moreover, such choice should fall within the scope of abilities of those skilled in the art.

### POLY(AMINO ACID) FOR PROTEIN (CYCLIZATION) CONJUGATION

An embodiment of the disclosure provides a poly(amino acid) for protein (cyclization) conjugation, having a structure represented by general formula 5: 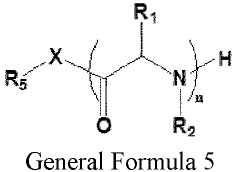 wherein
X represents sulphur or selenium;
R₁ independently represents, at each occurrence, a side chain of a non-natural amino acid;
R₂ independently represents, at each occurrence, hydrogen or C₁-C₁₀ alkyl, C₂-C₁₀ alkenyl, C₂-C₁₀ alkynyl;
R₅ represents substituted or unsubstituted C₁-C₃₀ alkyl, substituted or unsubstituted C₂-C₃₀ alkenyl, substituted or unsubstituted C₂-C₃₀ alkynyl, substituted or unsubstituted C₃-C₃₀ cycloalkyl, substituted or unsubstituted C₃-C₃₀ cycloalkenyl, substituted or unsubstituted C₁-C₃₀ heteroalkyl, substituted or unsubstituted C₂-C₃₀ heteroalkenyl, substituted or unsubstituted C₂-C₃₀ heteroalkynyl, substituted or unsubstituted C₁-C₃₀ heterocycloalkyl, substituted or unsubstituted C₂-C₃₀ heterocycloalkenyl, substituted or unsubstituted C₆-C₃₀ aryl, or substituted or unsubstituted C₅-C₃₀ heteroaryl; and
n is an integer selected from 1 to 200.

According to an embodiment of the disclosure, R₁ represents, at each occurrence, a side chain of a non-natural amino acid, where the corresponding natural amino acid is selected from the group consisting of glycine, alanine, valine, leucine, isoleucine, proline, phenylalanine, tyrosine, tryptophan, serine, threonine, cysteine, methionine, aspartic acid, glutamic acid, lysine, arginine and histidine. According to this embodiment of the disclosure, R₁ represents, at each occurrence, a side chain of a non-natural amino acid, such as those obtained from artificially modifying the above natural amino acid. According to yet another embodiment of the disclosure, R₁ represents, at each occurrence, a side chain of tyrosine, serine, threonine, cysteine, aspartic acid and/or glutamic acid that has been modified with oligo(ethylene glycol) (polymerization degree: 2-10, such as triethylene glycol, OEG₃), phosphate, propenyloxybenzyl ester or allyl triethylene glycol. According to other embodiment of the disclosure, R₁ represents, at each occurrence, a side chain of triethylene glycol (OEG₃) modified tyrosine, serine, threonine, cysteine, aspartic acid and/or glutamic acid.

According to an embodiment of the disclosure, R₂ represents, at each occurrence, hydrogen or C₁-C₁₀ alkyl. According to another embodiment of the disclosure, R₂ represents, at each occurrence, hydrogen, methyl, ethyl, n-propyl, isopropyl, n-butyl, tert-butyl, pentyl, neopentyl or hexyl. According to yet another embodiment of the disclosure, R₂ represents hydrogen.

According to an embodiment of the disclosure, R₅ represents substituted or unsubstituted C₁-C₁₀ alkyl, substituted or unsubstituted C₂-C₁₀ alkenyl, substituted or unsubstituted C₂-C₁₀ alkynyl, substituted or unsubstituted C₃-C₁₀ cycloalkyl, substituted or unsubstituted C₃-C₁₀ cycloalkenyl, substituted or unsubstituted C₁-C₁₀ heteroalkyl, substituted or unsubstituted C₂-C₁₀ heteroalkenyl, substituted or unsubstituted C₁-C₁₀ heterocycloalkyl, substituted or unsubstituted C₂-C₁₀ heterocycloalkenyl, substituted or unsubstituted C₆-C₁₈ aryl, or substituted or unsubstituted C₅-C₁₈ heteroaryl. According to another embodiment of the disclosure, R₅ represents substituted or unsubstituted C₁-C₁₀ alkyl, substituted or unsubstituted C₃-C₁₀ cycloalkyl, substituted or unsubstituted C₁-C₁₀ heteroalkyl, substituted or unsubstituted C₁-C₁₀ heterocycloalkyl, substituted or unsubstituted C₆-C₁₈ aryl, or substituted or unsubstituted C₅-C₁₈ heteroaryl.

According to an embodiment of the disclosure, a substituent for substituting R₅ is selected from the group consisting of C₁₋₃ haloalkyl, hydroxy, amino, mercapto, carbonyl, carboxy, sulfo, carboxylate, sulfonate, ester group, amide and/or halogen. According to another embodiment of the disclosure, R₅ is selected from, but is not limited to, the following structural formulae:

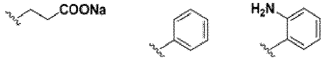

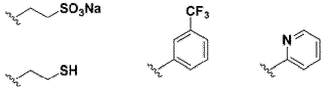

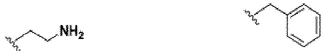

According to yet another embodiment of the disclosure, R₅ represents methyl, ethyl, propyl, n-butyl, tert-butyl, pentyl, hexyl, cyclopropyl, cyclobutyl, cyclopentyl, cyclohexyl or phenyl. According to other embodiment of the disclosure, R₅ represents phenyl.

According to an embodiment of the disclosure, n represents an integer selected from 1 to 200. According to another embodiment of the disclosure, n represents an integer selected from 1 to 150. According to yet another embodiment of the disclosure, n represents an integer selected from 1 to 100. According to other embodiment of the disclosure, n represents an integer selected from 1 to 50. In theory, n may be any numerical value as long as the resulting poly(amino acid) stably exists and falls within the scope of the claims, and can be specifically set by those skilled in the art based on practical application. Moreover, such choice should fall within the scope of abilities of those skilled in the art.

An embodiment of the disclosure provides a poly(amino acid) for protein (cyclization) conjugation, having a structure represented by general formula 6: 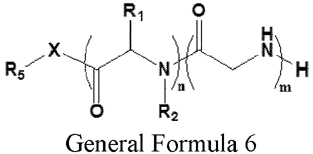 wherein
X, R₁, R₂ and R₅ are as defined in general formula 5;
n is an integer selected from 10 to 200; and
m is an integer selected from 1 to 30.

According to an embodiment of the disclosure, n represents an integer selected from 10 to 150. According to another embodiment of the disclosure, n represents an integer selected from 10 to 120. According to yet another embodiment of the disclosure, n represents an integer selected from 10 to 100. According to other embodiment of the disclosure, n represents an integer selected from 10 to 80. According to other embodiment of the disclosure, n represents an integer selected from 10 to 50. According to an embodiment of the disclosure, m represents an integer selected from 1 to 20. According to another embodiment of the disclosure, m represents an integer selected from 1 to 10. As mentioned above, n and m may be any numerical value, provided that the resulting conjugate may stably exist and falls within the scope of the claims. n and m can be specifically set by those skilled in the art based on practical application. Moreover, such choice should fall within the scope of abilities of those skilled in the art.

### METHOD FOR PREPARING PROTEIN-POLY(AMINO ACID) CONJUGATE

An embodiment of the disclosure provides a method for preparing the protein-poly(amino acid) conjugate as described herein, comprising: (1) initiating polymerization of a N-carboxyanhydride by an initiator R₅XY to obtain a poly(amino acid) having a carbon structure; and (2) mixing the poly(amino acid) with a protein containing a 1,2-mercaptoethylamine structure to obtain a protein-poly(amino acid) conjugate through native chemical ligation wherein X represents sulphur or selenium; Y represents hydrogen or trialkylsilyl, wherein the alkyl moiety in the trialkylsilyl is C₁-C₁₀ alkyl; and R₅ represents substituted or unsubstituted C₁-C₃₀ alkyl, substituted or unsubstituted C₂-C₃₀ alkenyl, substituted or unsubstituted C₂-C₃₀ alkynyl, substituted or unsubstituted C₃-C₃₀ cycloalkyl, substituted or unsubstituted C₃-C₃₀ cycloalkenyl, substituted or unsubstituted C₁-C₃₀ heteroalkyl, substituted or unsubstituted C₂-C₃₀ heteroalkenyl, substituted or unsubstituted C₂-C₃₀ heteroalkynyl, substituted or unsubstituted C₁-C₃₀ heterocycloalkyl, substituted or unsubstituted C₂-C₃₀ heterocycloalkenyl, substituted or unsubstituted C₆-C₃₀ aryl, or substituted or unsubstituted C₅-C₃₀ heteroaryl.

According to an embodiment of the disclosure, the step (1) comprises: initiating polymerization of a N-carboxyanhydride by an initiator (R₅XY) to obtain a poly(amino acid) (PAA-XR₅) containing a carbon structure, as shown below: 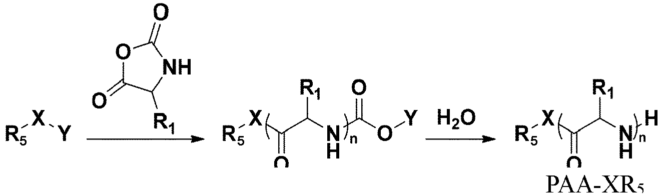 wherein
X, R₁, R₅ and n are as defined in general formula 5;
Y represents hydrogen or trialkylsilyl, where the alkyl moiety in the trialkylsilyl is preferably C₁-C₁₀ alkyl, such as methyl, ethyl, propyl, butyl, pentyl, hexyl and the like.

According to another embodiment of the disclosure, if Y is a trialkylsilyl, the trialkylsilyl carbamate structure at the N-terminus of the resulting poly(amino acid) tends to be removed when encountering with moisture in air, so that the N-terminus of the finally resulting poly(amino acid) is amino.

According to yet another embodiment of the disclosure, the step (1) comprises: initiating polymerization of a N-carboxyanhydride by an initiator (R₅XY) to obtain a poly(amino acid) (PAA-XR₅) containing a carbon structure, as shown below: 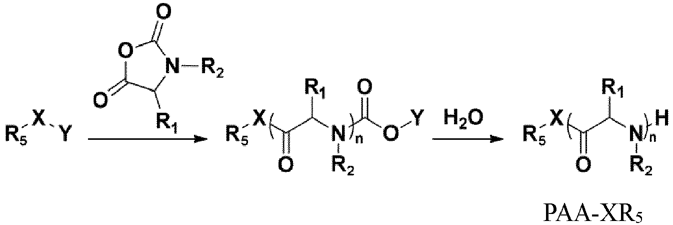 wherein
X, R₁, R₂, R₅ and n are as defined in general formula 5;
Y represents hydrogen or trialkylsilyl, where the alkyl moiety in the trialkylsilyl is preferably C₁-C₁₀ alkyl, such as methyl, ethyl, propyl, butyl, pentyl, hexyl and the like.

According to an embodiment of the disclosure, taking R₅XY being trimethylsilyl benzenethiol or trimethylsilyl benzeneselenol as an example, the reaction equation is: 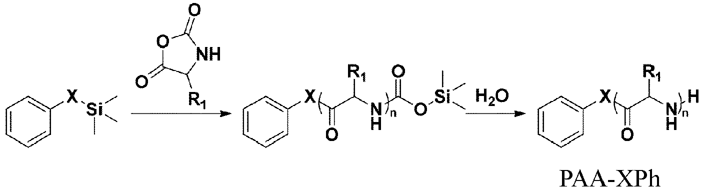 where X, R₁ and n are as defined in general formula 5.

According to an embodiment of the disclosure, in the step (1), polymerization is carried out in an aprotic solvent, such as dimethylformamide (DMF), tetrahydrofuran (THF) or dichloromethane (DCM). According to another embodiment of the disclosure, the reaction temperature is usually room temperature (25 °C), and the reaction duration depends on the monomer and the desired polymerization degree, ranging from dozens of minutes to dozens of hours, such as 20 minutes, 40 minutes, 1 hour, 10 hours, 15 hours, 20 hours, 25 hours and 30 hours.

According to an embodiment of the disclosure, a poly(amino acid) containing a carbon structure obtained in the step (1) may be subjected to post-modification, comprising: mixing and reacting the poly(amino acid) with a modifier to obtain a post-modified poly(amino acid). According to another embodiment of the disclosure, the modifier is mercaptoethylamine hydrochloride or mercaptopropionic acid. According to yet another embodiment of the disclosure, the post-modification process may last for a period of time under exposure to an ultraviolet lamp, e.g., for 1 hour, 3 hours, 5 hours, 10 hours, etc., which may be determined by those skilled in the art based on specific reaction progress. According to other embodiments of the disclosure, the post-modification is carried out in a solution, such as a solution containing an aprotic solvent, e.g., dimethylformamide (DMF), tetrahydrofuran (THF) or dichloromethane (DCM), and the solution may further contain a photosensitizer to facilitate the reaction, such as benzoin dimethyl ether (DMPA).

According to an embodiment of the disclosure, the step (2) comprises: mixing the poly(amino acid) obtained in the step (1) with a protein containing a 1,2-mercaptoethylamine structure (e.g., one having a cysteine at the N-terminus, or a non-natural amino acid containing a 1,2-mercaptoethylamine structure inserted into the C-terminus or any other site) to obtain a site-specific protein-poly(amino acid) conjugate through native chemical ligation at room temperature, as shown below: 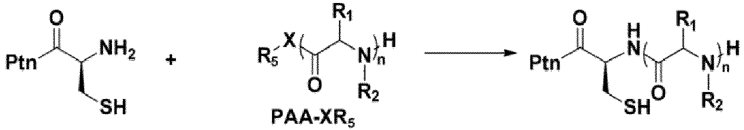 where Ptn, R₁, R₂ and n are as defined in general formula 1.

According to another embodiment of the disclosure, the step (2) comprises: mixing the poly(amino acid) obtained in the step (1) with a protein containing a 1,2-mercaptoethylamine structure (e.g., one having a cysteine at the N-terminus, or a non-natural amino acid containing a 1,2-mercaptoethylamine structure inserted into the C-terminus or any other site) to obtain a site-specific protein-poly(amino acid) conjugate through native chemical ligation at room temperature, as shown below: 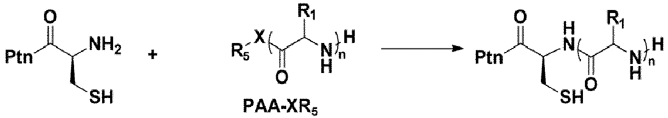 where Ptn, R₁ and n are defined in general formula 1.

According to an embodiment of the disclosure, a reaction solution at pH 6.5-7.0 is used in the step (2). A reductant TCEP (tris(2-carboxyethyl)phosphine) will be usually added to the reaction solution, but it is not necessary.

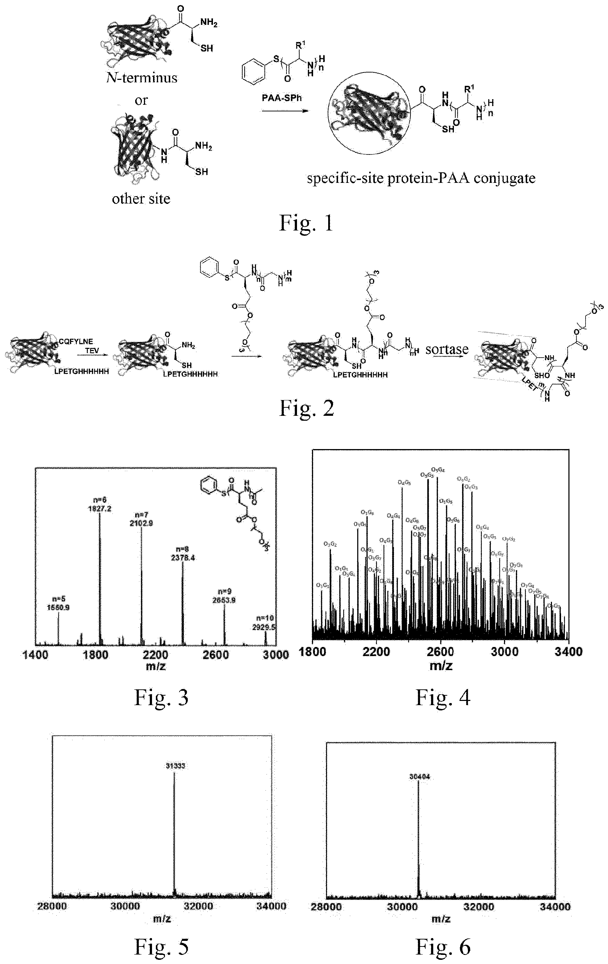

The method comprises initiating polymerization of a N-carboxyanhydride by an initiator R₅XY to obtain a poly(amino acid) PAA-XR₅ containing a carbon structure, and mixing PAA-XR₅ with a protein containing a 1,2-mercaptoethylamine structure to obtain a site-specific protein-poly(amino acid) conjugate through native chemical ligation at room temperature. Taking initiating polymerization of N-carboxyanhydride by trimethylsilyl benzenethiol as an example, the process is shown in Fig. 1.

According to a standard molecular cloning process, a cysteine-glycine may be inserted into the N-terminus of a protein (the N-terminus is cysteine residue, and glycine residue is used as a connection to increase the flexibility of the N-terminus). Alternatively, a non-natural amino acid with 1,2-mercaptoethylamine structure in its side chain is inserted using TAG and succinyl aminoacyl-tRNA synthetase/tRNA method (see Nguyen, D. P.; Elliott, T.; Holt, M.; Muir, T. W.; Chin, J. W., Genetically encoded 1,2-aminothiols facilitate rapid and site-specific protein labeling via a bio-orthogonal cyanobenzothiazole condensation. J Am Chem Soc 2011, 133 (30), 11418-21) for subsequent site-specific protein-poly(amino acid) conjugation reaction.

### METHOD FOR PREPARING PROTEIN-POLY(AMINO ACID) CYCLIC CONJUGATE

An embodiment of the disclosure provides a method for preparing the protein-poly(amino acid) cyclic conjugate as described herein, comprising: (1) initiating polymerization of a N-carboxyanhydride and a glycine N-carboxyanhydride by an initiator R₅XY to obtain a block poly(amino acid) having a phenylthioester structure at the C-terminus and a block polyglycine structure at the N-terminus; and (2) mixing the block poly(amino acid) with a protein having a cysteine at the N-terminus and a LPXaTG sequence at the C-terminus to successively conduct native chemical ligation and sortase mediated ligation to achieve cyclization of the protein, wherein the LPXaTG sequence is a recognition site of the sortase, wherein X represents sulphur; Y represents hydrogen or trialkylsilyl, wherein the alkyl moiety in the trialkylsilyl is C₁-C₁₀ alkyl; and R₅ represents phenyl group.

According to an embodiment of the disclosure, the step (1) comprises: initiating polymerization of a N-carboxyanhydride and a glycine N-carboxyanhydride by an initiator (R₅XY) to obtain a block poly(amino acid) having a phenylthioester structure at the C-terminus and a block polyglycine structure at the N-terminus, as shown below: 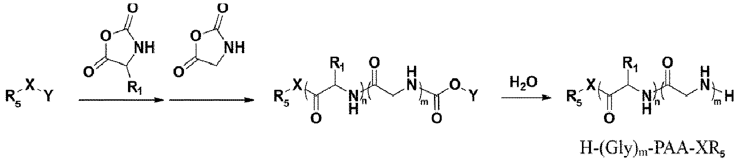 where R₁, R₅, X, Y, m and n are as defined in general formula 6.

According to another embodiment of the disclosure, the step (1) comprises: initiating polymerization of a N-carboxyanhydride and another N-carboxyanhydride by an initiator (R₅XY) to obtain a block poly(amino acid) having a phenylthioester structure at the C-terminus and a block polyglycine structure at the N-terminus, as shown below: 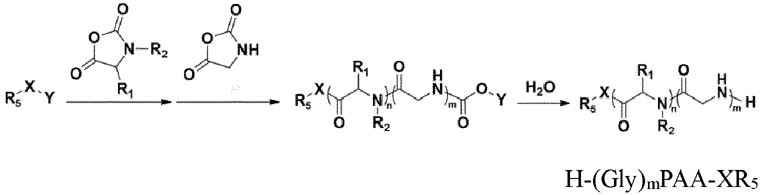 where X, R₁, R₂, R₅, n and m are as defined in general formula 6.

Y represents hydrogen or trialkylsilyl, where the alkyl moiety in the trialkylsilyl is preferably C₁-C₁₀ alkyl, such as methyl, ethyl, propyl, butyl, pentyl, hexyl and the like.

According to an embodiment of the disclosure, the step (2) comprises: mixing the block poly(amino acid) (e.g., H-(Gly)ₘPAA-XR₅) obtained in the step (1) with a protein having a cysteine at the N-terminus and a LPXaTG (Xa is any amino acid) sequence at the C-terminus to successively conduct native chemical ligation and sortase mediated ligation to achieve cyclization of the protein, as shown below:

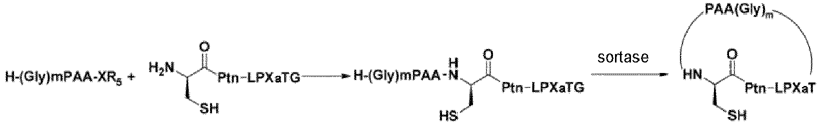

The LPXaTG sequence is a recognition site of the sortase, and the cyclization is achieved through ligation reaction between the block poly(amino acid) and the cysteine at the N-terminus of the protein, and then sortase mediated ligation. In order to facilitate subsequent protein purification, a polyhistidine tag (His-tag) can be added to the C-terminus of the protein before reaction, and then the protein is purified by nickel affinity chromatography after cyclization.

According to an embodiment of the disclosure, by plasmid construction, a sequence ENLYFQ digested by tobacco etch virus protease (TEV) is inserted into the N-terminus of eGFP, and a LPETG sequence is inserted into the C-terminus of the eGFP, for TEV-catalyzed conjugation. Specific amino acid sequence of a protein is shown in SEQ ID No: 1 in the sequence list. The C-terminus of the block poly(amino acid) is ligated with a protein having a cysteine at the N-terminus through native chemical ligation, and its N-terminus is ligated with the LPETG at the C-terminus of the protein through sortase mediated ligation. The reaction is shown in Fig. 2.

The amino acid sequence obtained from the polymerization according to the disclosure may consist of any water-soluble amino acid, including a natural amino acid, a non-natural amino acid, a L-amino acid, a D-amino acid, etc., and therefore has a significant advantage of material diversity in terms of biological expression. Moreover, the α-poly(amino acid) obtained from the polymerization according to the disclosure has a controllable molecular weight and molecular weight distribution. Therefore, polypeptide materials of different lengths may be prepared based on actual needs. The method according to the disclosure could in situ produce a thioester at the C-terminus of and a repeated glycine sequence at the N-terminus of the α-poly(amino acid), and the poly(amino acid) can be connected with proteins through a very efficient reaction, thereby greatly reducing the cost of protein modification and providing a broad application prospect.

The protein-polymer conjugate according to the disclosure inherits the advantages of traditional protein-polymer conjugate, for example, connecting a polymer having special properties (e.g., thermal response, photo-response or the like) to a protein to enable the protein-polymer conjugate to have certain functions and responsive properties. In addition, the degradation of a protein usually occurs at the C-terminus or the N-terminus. Therefore, the cyclization by connecting both termini with a polymer chain may reduce the protein degradation rate, increase its stability, and extend its blood circulation time. In view of the above, the disclosure provides a very wide application prospect for proteins.

### EXAMPLES

The following examples facilitate better understanding of the disclosure, and are not intended to limit the disclosure in any way. Unless otherwise specifically indicated, the test methods used in the following examples are conventional methods. Unless otherwise specifically indicated, the materials, reagents, etc. used in the following examples are commercially available reagents and materials. The plasmids are obtained through standard molecular cloning method.

### Example 1: Synthesis of Poly(amino acid) P1 for Protein Conjugation

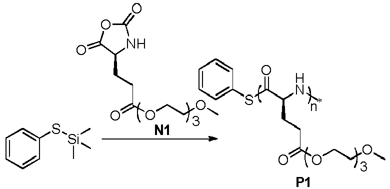

In a glove box, L-glutamate tris(ethylene glycol)-N-carboxyanhydride (N1, 40.0 mg, 0.131 mmol, 20 equiv) was dissolved in anhydrous N,N-dimethylformamide (1.0 mL), and a solution of trimethylsilyl benzenethiol in DMF (13.6 µL × 0.5 M, 1.0 equiv) was added thereto. After stirring at room temperature for 25h, the reaction mixture was poured into 40 mL of ethyl ether solution, and the resulting white precipitate was the product. A solid product was obtained through centrifugation. After discarding the supernatant, the residue was further washed with 40 ml of ethyl ether, and centrifuged to obtain a solid. After discarding the supernatant, 30 mg of colorless transparent colloidal solid was obtained through drying in a vacuum oven (yield: 72%), and was kept in a refrigerator at -20 °C.

L-glutamate tris(ethylene glycol) with an average polymerization degree of 7 was synthesized using a similar method, except that the addition amount of L-glutamate tris(ethylene glycol)-N-carboxyanhydride was 13 mg, and after the reaction, acetic anhydride (1.33 mg, 2.0 equiv) was added for end capping. After post-processing with the above method, characterization by MALDI-TOF-MS is shown in Fig. 3.

### Example 2: Synthesis of Block Poly(amino acid) P2 for Protein Cyclization

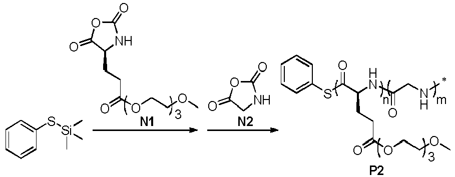

In a glove box, L-glutamate tris(ethylene glycol)-N-carboxyanhydride (N1, 13.0 mg, 0.044 mmol, 7 equiv) was dissolved in anhydrous N,N-dimethylformamide (1.0 mL), and a solution of trimethylsilyl benzenethiol in DMF (13.1 µL × 0.5 M, 1.0 equiv) was added thereto. After stirring at room temperature for 25h, glycine-N-carboxyanhydride (N2, 2.0 mg, 0.0197mmol, 3 equiv) was added, and the mixture was further stirred at room temperature for 2h. The post-processing method was identical to that in Example 1.

### Example 3: Synthesis of Poly(amino acid) P4 for Protein Conjugation

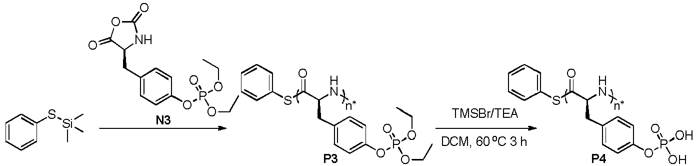

In a glove box, ethyl-L-tyrosine phosphate N-carboxyanhydride (N3, 100 mg, 0.291 mmol, 20 equiv) was dissolved in anhydrous N,N-dimethylformamide (2.0 mL), and a solution of trimethylsilyl benzenethiol in DMF (29.1 µL × 0.5 M, 1.0 equiv) was added thereto. The mixture was stirred at room temperature for 25h, and the post-processing method was identical to that in Example 1.

The poly(amino acid) (P3, 80 mg, 0.0133 mmol, 1.0 equiv) was dissolved in dichloromethane (1.5 mL), and triethylamine (300 µL, 2.1 mmol) and trimethylbromosilane (360 µL, 2.7 mmol) were successively added thereto. The reaction mixture was heated to 60 °C and stirred for 8 h. After rotary drying the solvent in a rotary evaporator, the product was dissolved in deionized water (4 mL), and EDTA (10 mg) was added thereto. The resulting solution was adjusted with aqueous solution of sodium hydroxide (1M) to a neutral pH, and dialyzed with aqueous solution of sodium chloride (1000 Da MWCO, 3 times). The solution was lyophilized to obtain 60mg of a light yellow solid P4 (yield: 83%).

### Example 4: Synthesis of Poly(amino acid) P5 for Protein Conjugation

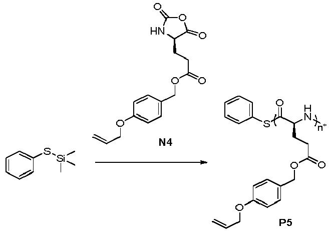

In a glove box, L-glutamate-4-propenyloxybenzyl-N-carboxyanhydride (N4, 300 mg, 0.952 mmol, 50 equiv) was dissolved in anhydrous N,N-dimethylformamide (3.0 mL), and a solution of trimethylsilyl benzenethiol in DMF (38.0 µL × 0.5 M, 1.0 equiv) was added thereto. The mixture was stirred at room temperature for 25h, and the post-processing method was identical to that in Example 1. Finally, 200 mg of colorless transparent colloidal solid was obtained (yield: 69%).

### Example 5: Synthesis of Poly(amino acid) P6 for Protein Conjugation

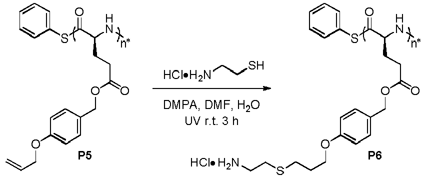

A poly(amino acid) (P5, 50 mg, 0.173 mmol, 1.0 equiv, calculated by the amount of substance having double bond) prepared in Example 4 was dissolved in N,N-dimethylformamide (2.0 mL); and mercaptoethylamine hydrochloride (118 mg, 1.04 mmol, 6.0 equiv) was dissolved in deionized water (200 µL); benzoin dimethyl ether (0.89 mg, 3.46×10⁻³ mmol, 0.02 equiv) was added to a mixture of the above two solutions, fully shaken, and then exposed to an ultraviolet lamp at 365 nm for 3 h. Upon completion of the reaction, the solution was poured into isopropanol (40 mL), and centrifuged to collect the precipitate. 32 mg of colorless, transparent and viscous colloidal solid was obtained through drying in a vacuum oven (yield: 53%).

### Example 6: Synthesis of Poly(amino acid) P7 for Protein Conjugation

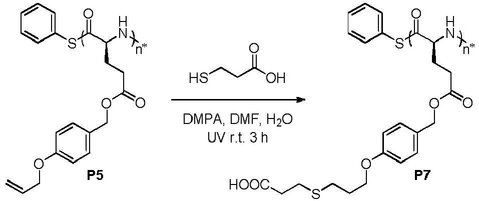

A poly(amino acid) (P1, 50 mg, 0.173 mmol, 1.0 equiv, calculated by the amount of substance having double bond) prepared in Example 1 was dissolved in N,N-dimethylformamide (2.0 mL); and mercaptopropionic acid (110 mg, 1.04 mmol, 6.0 equiv) was dissolved in deionized water (200 µL); benzoin dimethyl ether (0.89 mg, 3.46×10⁻³ mmol, 0.02 equiv) was added to a mixture of the above two solutions, fully shaken, and then exposed to an ultraviolet lamp at 365 nm for 3 h. The post-processing method was identical to that in Example 5. Finally, 33 mg of light yellow transparent colloidal solid was obtained (yield: 51%).

### Example 7: Synthesis of Poly(amino acid) P8 for Protein Conjugation

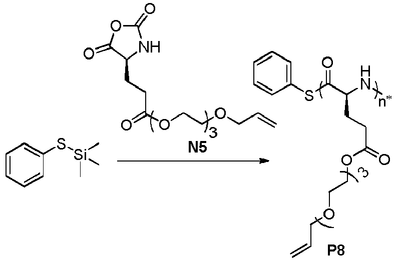

In a glove box, L-glutamate-propenyl tris(ethylene glycol)-N-carboxyanhydride (N5, 200 mg, 0.580 mmol, 50 equiv) was dissolved in anhydrous N,N-dimethylformamide (2.0 mL), and a solution of trimethylsilyl benzenethiol in DMF (23.2 µL × 0.5 M, 1.0 equiv) was added thereto. The mixture was stirred at room temperature for 25h, and the post-processing method was identical to that in Example 1. Finally, 160 mg of colorless transparent colloidal solid was obtained (yield: 82%).

### Example 8: Post-Modification of Poly(amino acid) for Protein Conjugation

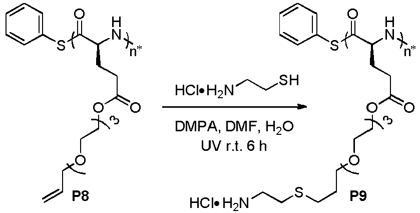

A poly(amino acid) (P8, 50 mg, 0.155 mmol, 1.0 equiv, calculated by the amount of substance having double bond) prepared in Example 7 was dissolved in N,N-dimethylformamide (2.0 mL); and mercaptoethylamine hydrochloride (113 mg, 0.94 mmol, 6.0 equiv) was dissolved in deionized water (200 µL); benzoin dimethyl ether (0.84 mg, 3.30×10⁻³ mmol, 0.02 equiv) was added to a mixture of the above two solutions, fully shaken, and then exposed to an ultraviolet lamp at 365 nm for 6 h. Upon completion of the reaction, the solution was diluted with deionized water to 5 mL, treated with PD10 desalting column twice, and lyophilized to obtain 25 mg of colorless transparent colloidal solid (yield: 46%).

### Example 9: Synthesis of Poly(amino acid) P10 for Protein Conjugation

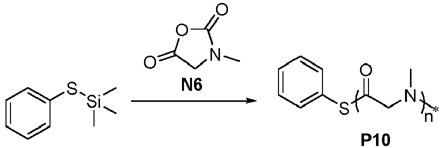

In a glove box, N-methyl-N-carboxyanhydride (N6, 15.0 mg, 0.131 mmol, 20 equiv) was dissolved in anhydrous N,N-dimethylformamide (1.0 mL), and a solution of trimethylsilyl benzenethiol in DMF (13.6 µL × 0.5 M, 1.0 equiv) was added thereto. The mixture was stirred at room temperature for 25h, and the post-processing method was identical to that in Example 1. Finally, 7 mg of a white solid was obtained (yield: 78%).

### Example 10: Synthesis of Block Poly(amino acid) P11 for Protein Conjugation

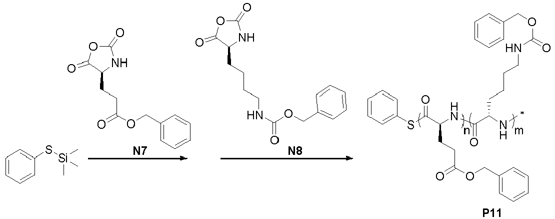

In a glove box, L-carbobenzoxy lysine-N-carboxyanhydride (N7, 100.3 mg, 0.328 mmol, 50 equiv) was dissolved in anhydrous N,N-dimethylformamide (1.0 mL), and a solution of trimethylsilyl benzenethiol in DMF (13.1 µL ×0.5 M, 1.0 equiv) was added thereto. After stirring at room temperature for 25h, benzyl L-glutamate N-carboxyanhydride (N8, 86.1 mg, 0.328 mmol, 50 equiv) was added, and the mixture was further stirred at room temperature for 25h. The post-processing method was identical to that in Example 1.

### Example 11: Synthesis of Poly(amino acid) P12 for Protein Conjugation

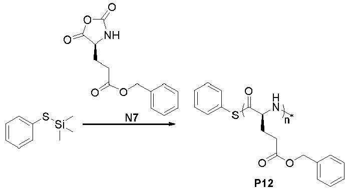

In a glove box, L-carbobenzoxy lysine-N-carboxyanhydride (N7, 100.3 mg, 0.328 mmol, 50 equiv) was dissolved in anhydrous tetrahydrofuran (1.0 mL), and a solution of trimethylsilyl benzenethiol in tetrahydrofuran (13.1 µL × 0.5 M, 1.0 equiv) was added thereto. The mixture was stirred at room temperature for 25h, and the post-processing method was identical to that in Example 1. Due to low initiation efficiency of the initiator in tetrahydrofuran, the actually obtained polymer had a high polymerization degree. The theoretical polymerization degree was 300.

### Example 12: Synthesis of Poly(amino acid) P13 for Protein Conjugation

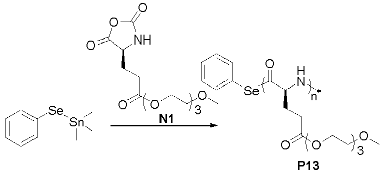

In a glove box, L-glutamate tris(ethylene glycol)-N-carboxyanhydride (N1, 13.0 mg, 0.131 mmol, 20 equiv) was dissolved in anhydrous N,N-dimethylformamide (1.0 mL), and a solution of trimethylstannyl benzeneselenol in DMF (13.6 µL ×0.5 M, 1.0 equiv) was added thereto. The mixture was stirred at room temperature for 25h, and the post-processing method was identical to that in Example 1.

### Example 13: Synthesis of Poly(amino acid) P14 for Protein Conjugation

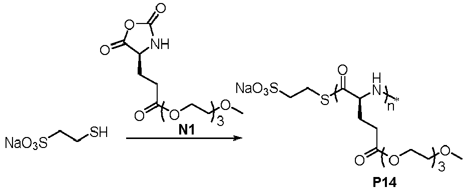

In a glove box, L-glutamate tris(ethylene glycol)-N-carboxyanhydride (N1, 13.0 mg, 0.131 mmol, 20 equiv) was dissolved in anhydrous N,N-dimethylformamide (1.0 mL), and a solution of sodium 2-mercaptoethanesulfonate in DMF (13.6 µL × 0.5 M, 1.0 equiv) was added thereto. The mixture was stirred at room temperature for 25h, and the post-processing method was identical to that in Example 1.

### Example 14: Synthesis of Poly(amino acid) P15 for Protein Conjugation

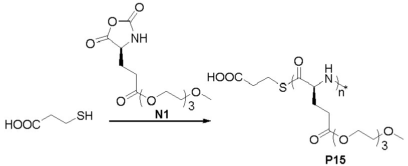

In a glove box, L-glutamate tris(ethylene glycol)-N-carboxyanhydride (N1, 13.0 mg, 0.131 mmol, 20 equiv) was dissolved in anhydrous N,N-dimethylformamide (1.0 mL), and a solution of mercaptopropionic acid in DMF (13.6 µL × 0.5 M, 1.0 equiv) was added thereto. The mixture was stirred at room temperature for 25h, and the post-processing method was identical to that in Example 1.

The characterization parameters of the polymers prepared in the above examples are shown in Table 1 below, and the values given below are averages of the measured values obtained from at least three measurements. Specifically, the molecular weight and polydispersity index (PDI) of all the polymers were measured by gel exclusion chromatography (GPC) in conjunction with angle light scatter, in which the mobile phase was anhydrous N,N -dimethylformamide containing 0.1 M lithium bromide.

**Table 1: Molecular Weight and Polydispersity Index of Polymers**

| Polymer No. | Theoretical polymerization degree | Actual polymerization degree | Actual molecular weight | PDI |
|---|---|---|---|---|
| P1 | 20 | 20 | 5600 | 1.12 |
| P2 | 10 | 9 | 2200 | 1.03 |
| P3 | 20 | 19 | 4700 | 1.05 |
| P5 | 50 | 52 | 17300 | 1.08 |
| P8 | 50 | 49 | 13500 | 1.06 |
| P10 | 20 | 20 | 1500 | 1.07 |
| P11 | 100 | 99 | 23900 | 1.03 |
| P12 | 300 | 305 | 67000 | 1.14 |
| P13 | 50 | 50 | 13900 | 1.08 |
| P14 | 50 | 48 | 13400 | 1.15 |
| P15 | 50 | 46 | 13000 | 1.06 |

As can be seen from the above table, the actual molecular weights of all the polymers are close to the theoretical molecular weights, and the molecular weight distribution is very narrow, indicating that the polymerization process is excellently controlled.

### Example 15: Preparation of Cyclic Conjugate of Polypeptide-Poly(amino acid) P2 (n=7, m=3)

The poly(amino acid) P2 (n=7, m=3) (3.8 mg, 1.72 µmol, 2.0 equiv) prepared in Example 2 and a polypeptide having a cysteine at the N-terminus (Cys-PEP, 1.8 mg, 0.86 µmol, 1.0 equiv, sequence CGDAKGLPETGHHHHHHK) were dissolved in ultra-pure water, adjusted with aqueous solution of sodium hydroxide (1.0 M) to pH 7.0, and reacted at room temperature for 12 h. After NiNTA affinity purification, the product was processed with a PD10 desalting column and lyophilized to obtain 2.6 mg of white powder, i.e., a native chemical ligation product (yield: 74%). The native chemical ligation product was dissolved in a Tris-HCl buffer solution (50 mM, 5.0 mL, pH 7.4) containing sodium chloride (150 mM) and calcium chloride (10 mM), and a sortase A (150 µL × 227 µM, 0.05 equiv) was added. After the mixture was kept at room temperature for 0.5 h, crude cyclic conjugate product polypeptide-poly(amino acid) was purified through NiNTA (eluted with a buffer solution B containing 20 mM Tris-HCl, 500 mM sodium chloride and 20mM imidazole, pH~8.0). The eluted components were collected and processed with a PD10 desalting column. 1.6 mg of white powder obtained through lyophilization was pure cyclic conjugate product (polypeptide-poly(amino acid) P2 (n=7, m=3)) (yield: 82%). The product was characterized through MALDI-TOF, and the molecular weight was consistent with the theoretical value, as shown in Fig. 4.

### Example 16: Expression and Purification of ENLYFQC-enhanced green fluorescent protein-LPETGH₆ (ENLYFQ-Cys-eGFP-LPETGH₆)

A plasmid pET-TEV-Cys-eGFP encoded with ENLYFQ at the N-terminus and LPETGHHHHHH at the C-terminus was transformed into E. coli BL21 by a chemical transformation method. The bacterial strain was revived from 10 mL of LB medium containing 100 µg/mL kanamycin for 10-12 h, then inoculated into 1 L of LB medium containing 100 µg/mL kanamycin at a density of 1:100, and shaken at 250 rpm at 37 °C until OD₆₀₀=0.8. The bacterial expression was induced by adding isopropyl thiogalactopyranoside at a final concentration of 1 mM, and the culture condition was changed to 250 rpm and 30 °C. 5 h later, bacteria were collected after centrifugation at 6500 rpm at 4 °C for 40 min. The bacterial was suspended in 20 mL of a buffer solution A (20 mM Tris-HCl, 500 mM NaCl, pH 8.0), and ultrasonically cleaved under an ice bath condition (130W, 20 kHz, 50% power, exposed to ultrasound for 5s, suspending for 10s, the time of ultrasound is 10 min). The lysate was successively centrifuged at 6500 rpm at 4 °C for 40 min and centrifuged at 12000 g at 4 °C for 40 min. The supernatant was collected, filtered through a 0.22µm filter membrane, and purified through a NiNTA affinity column, in which the equilibration buffer was 50 mM Tris containing 500 mM NaCl at pH 8.0, and the eluent was the equilibration solution supplemented with 300 mM imidazole. Finally, 48 mg of the target protein (sequence represented by SEQ ID No: 1 in the sequence list) was obtained, and identified by UPLC-MS (see Fig. 5). The calculated value is 31324, and the observed value is 31333. The molecular weight error (9 Da) falls within the range of allowable error (10 Da). Therefore, the obtained protein is identified as the target protein TEV-enhanced green fluorescent protein-LPETGH₆.

### Example 17: Expression of Site-Specific Enhanced Green Fluorescent Protein (eGFP) Containing 1,2-mercaptoethylamine Structure

### Step 1: Synthesis of Non-Natural Amino Acid Containing 1,2-mercaptoethylamine

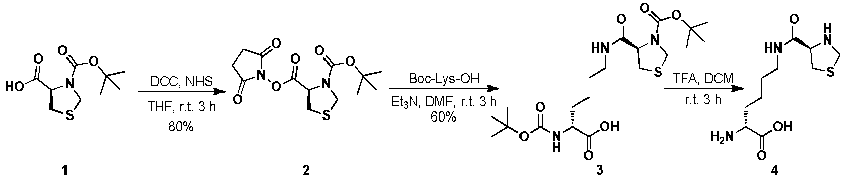

A compound 1 (233 mg, 1.00 mmol, 1.0 equiv) was dissolved in anhydrous tetrahydrofuran (20 mL), and N,N'-dicyclohexyl carbodiimide (DCC, 206 mg, 1.00 mmol, 1.0 equiv) and N-hydroxysuccinimide (NHS, 115 mg, 1.00 mmol, 1.0 equiv) were added thereto. The resulting solution is stirred at room temperature for 3 h, and filtered to remove solid. After rotary drying of the filtrate, the residue was separated through column chromatography, in which the eluting agent was dichloromethane: ethyl acetate=5:1 (volume ratio) to pure ethyl acetate. A white solid compound 2 (264 mg, yield: 80%) was obtained.

The compound 2 (264 mg, 0.80 mmol, 1.0 equiv) was dissolved in N,N-dimethylformamide (20 mL), and Boc-Lys-OH (246 mg, 0.80 mmol, 1.0 equiv) and triethylamine (0.2 mL) were added thereto. The resulting solution was stirred at room temperature for 3 h. After rotary drying of the solvent, the residue was separated through column chromatography, in which the eluting agent was dichloromethane to dichloromethane: methanol=10:1 (volume ratio). A colorless oily liquid compound 3 (221 mg, yield: 60%) was obtained.

The compound 3 (221 mg, 0.48 mmol, 1.0 equiv) was dissolved in a solution of hydrogen chloride in dioxane solution (10 mL), and stirred at room temperature overnight to obtain a precipitate. A product, i.e., compound 4 (non-natural amino acid containing 1,2-mercaptoethylamine) was obtained through filtration in vacuo, and was used for the follow-up experiment after pH adjustment.

### Step 2: Expression of Enhanced Green Fluorescent Protein

A plasmid having a specific site of a protein gene sequence mutated to a TAG (see the sequence list SEQ ID No: 2 (C-terminus His₆-tagged) for details of the original protein sequence; a codon corresponding to N of aspartic acid at the 149th site was mutated to a TAG to obtain a specific site-mutated gene sequence) and a plasmid carrying a succinyl aminoacyl-tRNA synthetase/tRNA were simultaneously transformed into E. coli BL21 by a chemical transformation method. The bacterial strain was revived from 10 mL of LB medium containing 100 µg/mL ampicillin and 34 µg/mL chloromycin for 10-12 h, then inoculated into 1 L of LB medium containing 100 µg/mL ampicillin and 34 µg/mL chloromycin, and incubated at 250 rpm at 37 °C for 2 h. A non-natural amino acid 4 was added at a final concentration of 2 mM, and shaken until OD₆₀₀=0.8. The bacterial expression was induced by adding isopropyl thiogalactopyranoside at a final concentration of 1 mM, and the culture condition was changed to 250 rpm and 30 °C. 12 h later, bacteria were collected after centrifugation at 6500 rpm at 4 °C for 40 min. The bacteria were suspended in 20 mL of a buffer solution A (20 mM tris-HCl, 500 mM NaCl, pH 8.0), and ultrasonically cleaved under an ice bath condition. The lysate was successively centrifuged at 6500 rpm at 4 °C for 40 min and centrifuged at 12000 g at 4 °C for 40 min. The supernatant was collected, filtered through a 0.22µm filter membrane, and purified with a NiNTA purification method similar to that in Example 16.

### Example 18: Enzymatic Digestion of ENLYFQC-Enhanced Green Fluorescent Protein-LPETGH₆ (ENLYFQ-Cys-eGFP-LPETGH₆)

1 mL of a protein solution (12 mg/mL), 5 µL of tobacco etch virus protease (0.2 mg/mL) and 10 µL of a 10X buffer solution B (500 mM sodium phosphate, 5 mm EDTA and 10 mM DTT) were added to a dialysis bag (MWCO 3000). The dialysis bag was placed in 1 L of a buffer solution B (50 mM sodium phosphate, 0.5 mM EDTA and 1 mM DTT) for dialysis and enzymatic digestion at room temperature. A protein Cys-eGFP-LPETGH₆ (its amino acid sequence was a sequence obtained by deleting "MENLYFQ" from the N-terminus of the SEQ ID No: 1 in the sequence list) was obtained through enzymatic digestion for 3 h, and was identified by UPLC-MS (see Fig. 6). The calculated value is 30398, and the observed value is 30404. The molecular weight error (6 Da) falls within the range of allowable error (10 Da). Thus the protein obtained through enzymatic digestion may be identified as the target protein Cys-green fluorescent protein-LPETGH₆.

### Example 19: Native Chemical Ligation between Cys-Enhanced Green Fluorescent Protein-LPETGH₆ and Poly(amino acid) P1 (N=7) (Protein N-Terminus Conjugation)

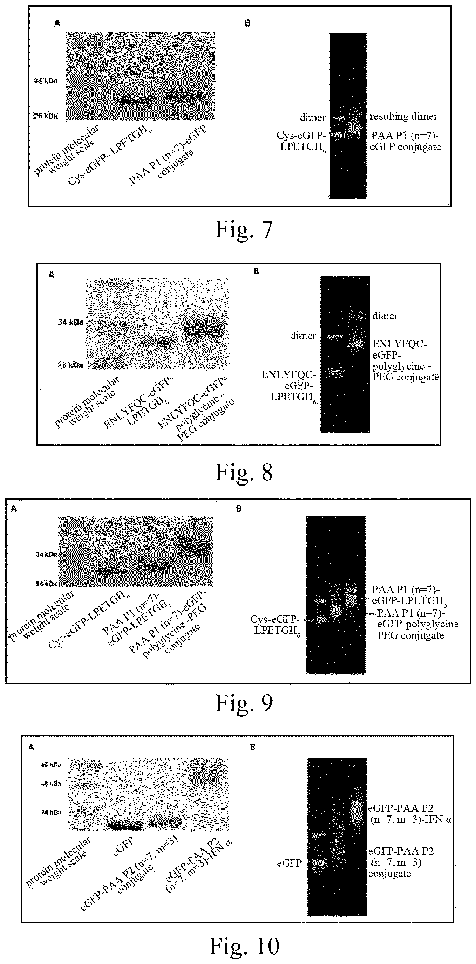

1 equiv of Cys-enhanced green fluorescent protein-LPETGH₆ and 2 equiv of poly(amino acid) P1 (n=7) were subjected to native chemical ligation in a solution of 50 mM Tris-HCl and 2 mM tris(2-carboxyethyl) phosphine at pH 6.5 for 10h, and the reaction product was purified by fast protein liquid chromatography (FPLC) to obtain a site-specific protein-polymer conjugate. Its molecular weight change was characterized through SDS-PAGE, and its purity was characterized through native PAGE. As shown in Fig. 7, in SDS-PAGE, the band of the poly(amino acid)-enhanced green fluorescent protein conjugate migrates to high molecular weight relative to the starting material protein, indicating that the poly(amino acid) is successfully grafted. The poly(amino acid)-enhanced green fluorescent protein conjugate has homogeneous single-bands in SDS-PAGE and native PAGE (the dimer is formed through physical interaction of the same monomer protein under a natural condition), suggesting that the product has a high purity, and its yield was 50%-65%.

### Example 20: Sortase Mediated Ligation (Protein C-Terminus Ligation) between ENLYFQC-Enhanced Green Fluorescent Protein-LPETGH₆ and Polyethylene Glycol-Polyglycine

1 equiv of an ENLYFQC-enhanced green fluorescent protein-LPETGH₆ (1 mg/ml, 300 µL), 5 equiv of a polyethylene glycol-polyglycine (10 mM, 53 µL) and 0.1 equiv of sortase (4.9 mg/mL, 52 µL) were added to 200 µL of a buffer solution of 50 mM Tris-HCl containing 150 mM NaCl and 10 mM CaCl₂ at pH 7.4. After reaction at room temperature for 30 min, the product ENLYFQC-enhanced green fluorescent protein-polyethylene glycol-polyglycine conjugate was separated through NiNTA affinity purification. The equilibration buffer was 50 mM Tris containing 500 mM NaCl at pH 8.0. The eluted buffer solution was collected since the sample injection, then the sample was eluted with 50 mM Tris containing 500 mM NaCl and 20 mM imidazole at pH 8.0 to obtain an eluent in which the product was dissolved, and then the resin was washed with a buffer solution of 50 mM Tris containing 500 mM NaCl and 300 mM imidazole at pH 8.0 to elute an un-reacted starting material protein. The molecular weight of the ENLYFQC-enhanced green fluorescent protein-polyethylene glycol-polyglycine conjugate was characterized through SDS-PAGE, and its purity was characterized through native PAGE. As shown in Fig. 8, in SDS-PAGE, the band of the ENLYFQC-enhanced green fluorescent protein-polyglycine-polyethylene glycol conjugate migrates to high molecular weight relative to the starting material protein, indicating that the poly(amino acid) is successfully grafted. The poly(amino acid)-enhanced green fluorescent protein conjugate has homogeneous single-bands in SDS-PAGE and native PAGE, suggesting that the product has a high purity, and its yield was 56%.

### Example 21: Preparation of Knot-like Conjugate of Poly(amino acid) P1 (n=7)-Enhanced Green Fluorescent Protein-Polyglycine-Polyethylene Glycol

A knot-like conjugate of poly(amino acid) P1 (n=7)-enhanced green fluorescent protein-polyglycine-polyethylene glycol was prepared in two steps successively according to the reaction and purification methods described in Examples 19 and 20. An intermediate product poly(amino acid) P1 (n=7)-enhanced green fluorescent protein-polyglycine-polyethylene glycol and a knot-like conjugate were characterized through both SDS-PAGE and native PAGE (see Fig. 9). The molecular weights of the products gradually migrate to high molecular weight of proteins, indicating that the polymer is successively successfully grafted, and a single band indicates that the product has high purity. The yields in the two steps were 65% and 50% respectively.

### Example 22: Preparation of Dumbbell-like Conjugate of Enhanced Green Fluorescent Protein-Poly(amino acid) P2 (n=7, m=3)-Interferon α

1 equiv of an enhanced green fluorescent protein-LPETGH₆ (6.5 mg), 10 equiv of a poly(amino acid) P2 (n=7, m=3) (6.6 mg) and 0.1 equiv of sortase (0.5 mg) were added to 1 mL of a Tris-HCl buffer solution (50 mM, pH7.5) containing 150 mM sodium chloride and 10 mM calcium chloride, and reacted at room temperature for 30 min. An enhanced green fluorescent protein-poly(amino acid) P2 (n=7, m=3) conjugate was obtained through purification process according to Example 21, and the yield was 55%. Then, the dumbbell-like conjugate of enhanced green fluorescent protein-poly(amino acid) P2 (n=7, m=3)-interferon α was obtained through native chemical ligation between the enhanced green fluorescent protein-poly(amino acid) P2 (n=7, m=3) conjugate and a Cys-IFN α according to Example 21, and the yield was 50%. The molecular weights and purities of the intermediate product and the dumbbell-like conjugate were characterized through SDS-PAGE and native PAGE (see Fig. 10). The molecular weights of the conjugates gradually migrate to high molecular weight, and the molecular weight of the dumbbell-like conjugate enhanced green fluorescent protein-poly(amino acid)-interferon α is distributed between 43 kDa and 55 kDa according to the protein molecular weight scale, which complies with the scope (~52kDa) expected by adding the enhanced green fluorescent protein (~31 kDa) with the interferon α (~21kDa). Therefore, it can be determined that the dumbbell-like conjugate enhanced green fluorescent protein-poly(amino acid)-interferon α was obtained. It can also be determined that the product has a high purity due to a single band.

### Example 23: Preparation of Cyclic Conjugate of Enhanced Green Fluorescent Protein-Poly(amino acid) P2 (n=7, m=3)

A poly(amino acid) P2 (n=7, m=3)-enhanced green fluorescent protein-LPETGH₆ conjugate was prepared using native chemical ligation according to Example 19. 1 equiv of the poly(amino acid) P2 (n=7, m=3)-enhanced green fluorescent protein-LPETGH₆ conjugate (0.42 mg) and 0.1 equiv of sortase were mixed in a 50 mM Tris-HCl buffer solution containing 150 mM sodium chloride and 10 mM calcium chloride at pH7.5. The final concentration of the poly(amino acid) P2 (n=7, m=3)-enhanced green fluorescent protein-LPETGH₆ conjugate was controlled to 50 mM. The mixture was reacted at room temperature for 30 min. Upon completion of the reaction, the product was separated and purified according to Example 22, and the yield was about 42%.

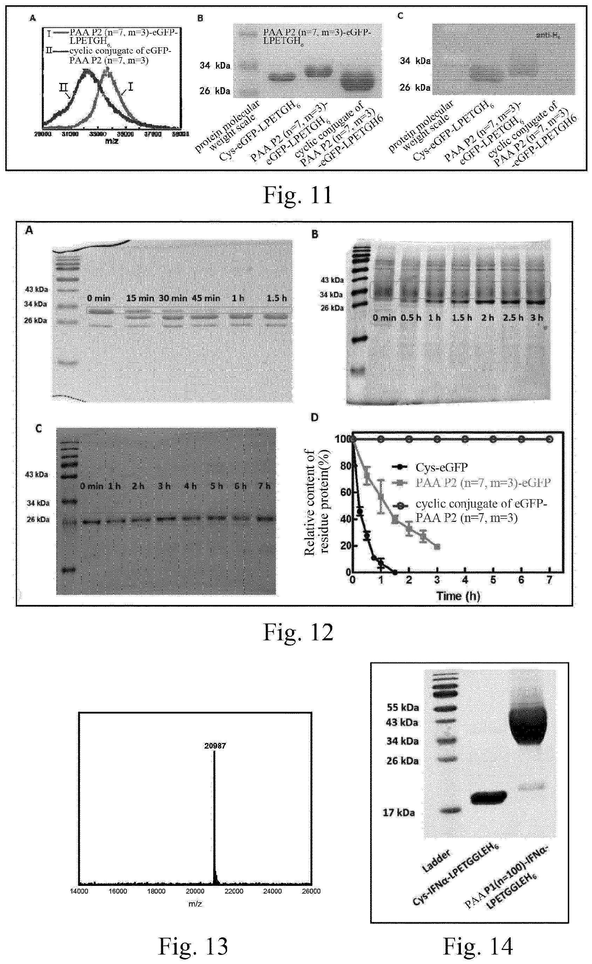

The cyclic conjugate of enhanced green fluorescent protein-poly(amino acid) P2 (n=7, m=3) was characterized using a matrix-assisted laser desorption ionization-time-of-flight mass spectrometer (MALDI-TOF), SDS-PAGE and western blot. The poly(amino acid) P2 (n=7, m=3)-enhanced green fluorescent protein-LPETGH₆ conjugate has 7 amino acids (i.e., GHHHHHH) at the carbon terminus more than the cyclic conjugate of enhanced green fluorescent protein-poly(amino acid) P2 (n=7, m=3). Therefore, according to MALDI-TOF analysis, the molecular weight of the cyclized poly(amino acid) P2 (n=7, m=3)-enhanced green fluorescent protein-LPETGH₆ conjugate is entirely lower than the molecular weight before cyclizatione (theoretically 1160 Da, actually about 1206 Da), directly indicating that the enhanced green fluorescent protein-poly(amino acid) P2 (n=7, m=3) forms a cyclic conjugate. Furthermore, in SDS-PAGE, the band of cyclic enhanced green fluorescent protein-poly(amino acid) P2 (n=7, m=3) will migrate to low molecular weight due to the decrease in molecular weight and the change in topological structure. Western blot analysis shows that His-tag disappears, further indicating that the cyclic conjugate of enhanced green fluorescent protein-poly(amino acid) P2 (n=7, m=3) is successfully obtained, and the specific analysis results are shown in Fig. 11.

### Example 24: Carboxypeptidase Stability Test of Cys-Enhanced Green Fluorescent Protein, Poly(amino acid) P2 (n=7, m=3)-Enhanced Green Fluorescent Protein and Cyclic Conjugate of Enhanced Green Fluorescent Protein-Poly(amino acid) P2 (n=7, m=3)

The final concentrations of a Cys-enhanced green fluorescent protein, a poly(amino acid) P2 (n=7, m=3)-enhanced green fluorescent protein and a cyclic conjugate of enhanced green fluorescent protein-poly(amino acid) P2 (n=7, m=3) in a 50 mM Tris-HCl buffer solution at pH7.4 were controlled to 4.0 mg/mL, and the final concentration of carboxypeptidase was controlled to 80 µg/mL with the equivalence ratio being 0.01 equiv of eGFP, and the incubation was carried out at 37 °C. At a set time point, 5 µL of a sample was taken and mixed with a buffer solution for sample injection, boiled at 95 °C for 10 min to terminate enzymatic digestion reaction. When sampling was completed at the last time point, all the samples were simultaneously subjected to gel electrophoresis, and characterized through protein gel electrophoresis. Upon completion of Coomassie brilliant blue staining and decoloration, a protein was quantified using a typhoon FLA laser scanner.

As shown in Fig. 12, Fig. A, B and C respectively show the carboxypeptidase mediated degradation of the Cys-enhanced green fluorescent protein, the poly(amino acid) P2 (n=7, m=3)-enhanced green fluorescent protein and the cyclic conjugate of enhanced green fluorescent protein-poly(amino acid) P2 (n=7, m=3) over time. The protein was quantified according to the intensity of the band after staining with a typhoon FLA laser scanner. At the same carboxypeptidase ratio, a wild type enhanced green fluorescent protein was substantively completely degraded within 1 h, while only about 20% of the complete conjugate of the poly(amino acid) P2 (n=7, m=3)-enhanced green fluorescent protein was left at 3 h. In contrast, a certain degree of degradation of the cyclic conjugate of enhanced green fluorescent protein-poly(amino acid) P2 (n=7, m=3) was observed since 7 h. Therefore, compared with the linear topological structure, the cyclic conjugate of enhanced green fluorescent protein-poly(amino acid) P2 (n=7, m=3) shows a great advantage in terms of enzymatic resistance.

### Example 25: Expression and Purification of Drug Protein ENLYFQCG-Interferon-LPETGLEH₆ (TEV-Cys-IFN α-LPETGLEH₆)

A plasmid pET-TEV-Cys-IFNα-LPETGLEH₆ encoded with MENLYFQCG at the N-terminus and LPETGLEHHHHHH at the C-terminus (SEQ ID No. 3) was transformed into E. coli OrigamiB (DE3) by a chemical transformation method. The bacterial strain was revived from 10 mL of LB medium containing 100 µg/mL ampicillin for 10-12 h, then inoculated into 1 L of LB medium containing 100 µg/mL ampicillin, and shaken at 250 rpm at 37 °C until OD₆₀₀=0.8. The bacterial expression was induced by adding isopropyl thiogalactopyranoside at a final concentration of 1 mM, and the culture condition was changed to 200 rpm and 30 °C. After overnight incubation, bacteria were collected by centrifugation at 6500 rpm at 4 °C for 30 min. The bacteria were resuspended in 20 mL of a buffer solution A (20 mM Tris-HCl, 500 mM NaCl, pH 8.0), and ultrasonically cleaved in an ice bath condition. The lysate was successively centrifuged at 6500 rpm at 4 °C for 40 min and centrifuged at 12000 g at 4 °C for 40 min. The supernatant was collected, filtered through a 0.22µm filter membrane, and purified through a NiNTA column to obtain 98 mg of the target protein, which was identified by UPLC-MS. The calculated theoretical molecular weight is 21918, and the observed value is 21914. The molecular weight error (4 Da) falls within the range of allowable error (10 Da). Therefore, the obtained protein can be identified as the target protein TEV-Cys-IFN α-LPETGLEH₆.

### Example 26: Enzymatic Digestion of Drug Protein ENLYFQCG-Interferon-LPETGLEH₆ (TEV-Cys-IFN α-LPETGLEH₆)

1 mL of a protein solution (12 mg/mL), 5 µL of tobacco etch virus protease (0.2 mg/mL) and 10 µL of a 10X buffer solution B (500 mM sodium phosphate, 5 mm EDTA and 10 mM DTT) were added to a dialysis bag (MWCO 3000). The dialysis bag was placed in 1 L of a buffer solution B (50 mM sodium phosphate, 0.5 mM EDTA and 1 mM DTT) for dialysis and enzymatic digestion at room temperature. A protein Cys-eGFP-LPETGLEH₆ was obtained through enzymatic digestion for 3 h, and was identified by UPLC-MS (see Fig. 13). The calculated value is 20991, and the observed value is 20987. The molecular weight error (6 Da) falls within the range of allowable error (10 Da). Therefore, the protein obtained through enzymatic digestion can be identified as the target protein Cys-IFN α-LPETGLEH₆.

### Example 27: Native Chemical Ligation between Drug Protein Cys-Interferon-LPETGLEH₆ and Poly(amino acid)s Having Different Lengths

1 equiv of the target protein Cys-IFNα-LPETGLEH₆ and 3 equiv of a poly(amino acid) PhS-P(OEG₃-Glu)₁₀₀ (L-type, P1 (n=100)) were subjected to native chemical ligation in a solution containing 50 mM Tris-HCl and 2 mM DTT at pH 7.4 for 10 h, the product was purified by fast protein liquid chromatography (FPLC) to obtain a site-specific P(OEG₃-L-Glu)₁₀₀-IFNα conjugate. The molecular weight and purity of the poly(amino acid) P1 (n=100)-IFNα conjugate were characterized through 15% SDS-PAGE. As shown in Fig. 14, in SDS-PAGE, the band of the poly(amino acid) P1 (n=100)-IFNα conjugate migrates to high molecular weight relative to the starting material protein, indicating that the poly(amino acid) is successfully grafted. The conjugate has homogeneous single-band, suggesting that the product has a high purity, and its yield was 70%.

Alternatively, a conjugate was obtained through native chemical ligation of the drug protein Cys-IFNα-LPETGLEH₆ with a poly(amino acid) PhS-P(OEG₃-Glu)₁₀₀ (D, L-amino acid) or a poly(amino acid) P2 (PhS-P(OEG₃-Glu)ₙ-Gly₃ (n=7 or 20, m=3)) using the above method and under the above conditions, purified by FPLC, and characterized through 15% SDS-PAGE. As shown in Fig. 14, the band of the poly(amino acid)-IFNa conjugate migrates to high molecular weight relative to the starting material protein, indicating that the poly(amino acid) is successfully grafted, and its yield was 70%-80%.

### Example 28: Site-Specific Conjugation of N- and C-Termini of Cys-IFNα-LPETG to Poly(amino acid)s of Different Lengths

The products poly(amino acid) P1 (n=100)-IFNα-LPETGLEH₆ in Example 27 and the poly(amino acid) P2 (n=20, m=3) were subjected to sortase mediated transpeptidation in a buffer solution containing 20 mM Tris-HCl, 150 mM NaCl and 10 mM CaCl₂, and the reaction product was separated and purified through a NiNTA column to obtain the product poly(amino acid) P1 (n=100)-IFNα-LPET-poly(amino acid) P2 (n=20, m=3), i.e., a conjugate in which both N- and C-termini were site-specifically conjugated to poly(amino acid)s. The product was characterized through 15% SDS-PAGE and western blot. In western blot analysis, as shown in Fig. 14, since the His-tag was removed, anti-His tag signal of the product disappeared, indicating that poly(amino acid)s were successfully grafted to both N- and C-termini of the IFN α.

### Example 29: Cyclization of Poly(amino acid) P2 (n=7 or 20, m=3)-IFNα-LPETGLEH₆ Conjugate

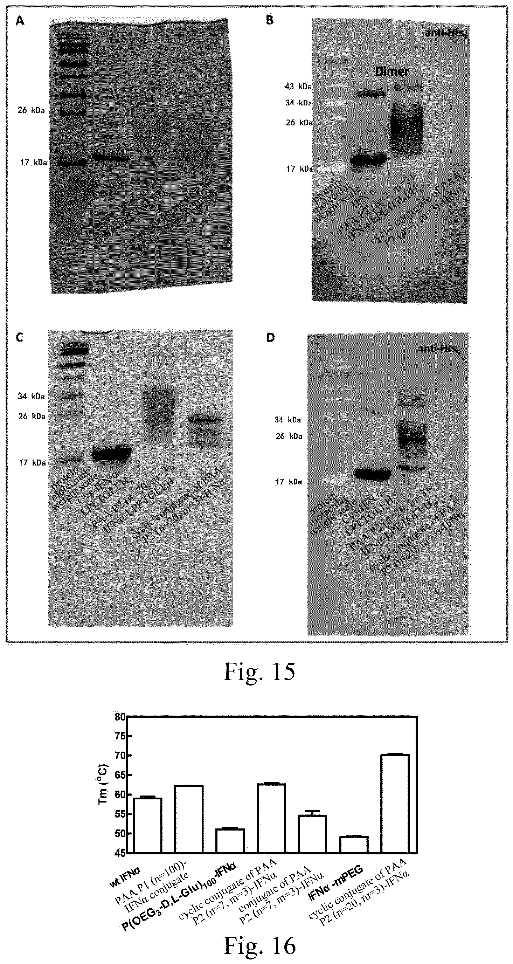

A poly(amino acid) P2 (n=7 or 20, m=3)-IFNα-LPETGLEH₆ conjugate was subjected to sortase mediated cyclization in a buffer solution containing 20 mM Tris-HCl, 150 mM NaCl and 10 mM CaCl₂, and the product was separated and purified through a NiNTA column to obtain cyclic conjugate of poly(amino acid) P2 (n=7 or 20, m=3)-IFNα-LPETGLEH₆. As shown in Fig. 15, the formation of a cyclic topological structure of an IFN α and a poly(amino acid) can be identified by the migration of the band to low molecular weight in SDS-PAGE relative to the conjugate before cyclization, and the disappearance of anti-His signals in corresponding western blot.

### Example 30: Thermal Stability Test of Poly(amino acid) P1 (n=100)-IFNα and Cyclic Conjugate of Poly(amino acid) P2 (n=7 or 20, m=3)-IFNα

In the example, the target protein was a recombinant human IFNα, and the protein melting temperature (Tm value) was measured with a dye Sypro Orange. The dye can bond to a hydrophobic domain that will be exposed with the protein conformation change caused by temperature rise, so that the fluorescence emission intensity is greatly changed. Tm values of a wild type IFN α and various conjugates of the wild type IFN α and poly(amino acid) were measured by followings. 5 µL of 200 × Sypro Orange (Thermo) was mixed with 45 µL (5 µM) of protein to be tested in 1×PBS buffer solution to obtain a total of 50 µL of a reaction system. It was added to a 96-well plate (with three parallel wells), detected with a fluorescent quantitative PCR, and heated to 98°C from 37°C at a rate of 2.2°C/min to calculate the Tm value of each protein sample based on the obtained curve.

The thermal stability of the conjugates can be concluded from the Tm values of the protein samples as shown in Fig. 16. The higher the Tm is, the better the thermal stability is. The poly(amino acid) P1 (n=100)-IFNα conjugate and the cyclic conjugate of poly(amino acid) P2 (n=7 or 20, m=3)-IFNα show very excellent thermal stability. In particular, the thermal stability of the cyclic conjugate of poly(amino acid) P2 (n=7 or 20, m=3)-IFNα is the best compared with those reported in existing literatures and patents.

### Example 31: Trypsin Resistance Test of Poly(amino acid)-IFN α Conjugates

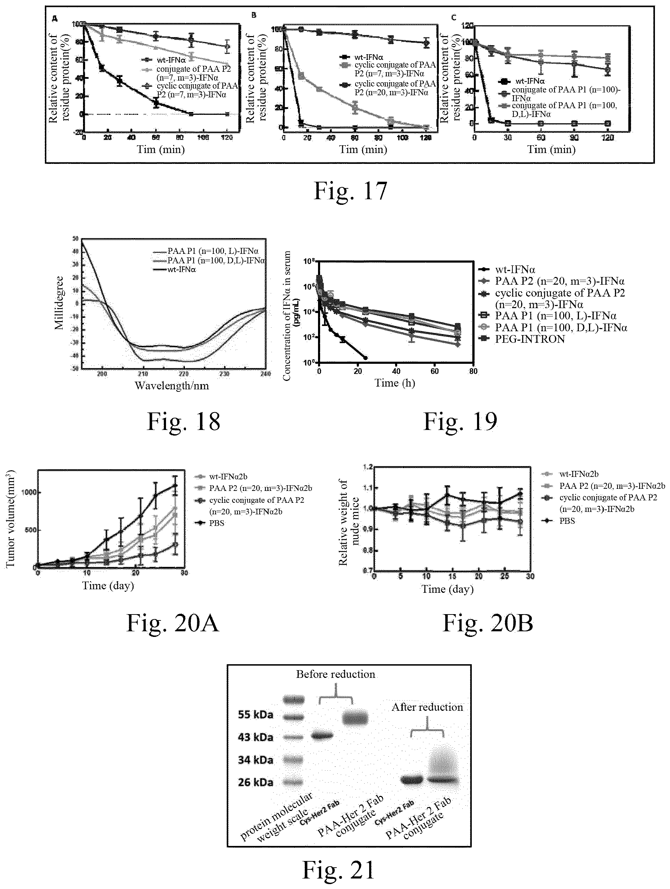

In the example, trypsin resistance of a poly(amino acid)-IFNα conjugate according to the disclosure was tested with a wild type recombinant human IFNα as the control. At 37 °C, 0.01 equiv of trypsin was added to treat samples, and the proportion of remaining intact protein was detected by sampling at different time points. The results were shown in Fig. 17, in which the graphs (as shown in Fig. 17) were drawn based on the quantitative data of the intensity of the protein band. As can be found from graphs B and C, with the increase of the molecular weight of the poly(amino acid) used in the conjugate, the trypsin resistance of the resulting conjugate is increased, and according to graph A, as for the conjugates with close molecular weights, trypsin resistance of the conjugate having a cyclic topological structure is significantly better than those of non-cyclized conjugate.

### Example 32: Circular Dichroism Test of Wild Type Interferon (wt-IFNα) and Poly(amino acid) P1 (n=100 L-type or n=100 D,L-type)-IFN α Conjugate

In the example, the used poly(amino acid) P(OEG₃-Glu)ₙ (L-type) separately had a secondary structure mainly being α-helix, and its ligation with an IFNα will have certain influence on the secondary structure of the IFNα. A protein was prepared into a PBS solution at 0.35 mg/mL to monitor the change of the secondary structure by circular dichroism, and the results show that an IFNα-poly(amino acid) conjugate has significantly enhanced the secondary structure of the IFNα, as shown in Fig. 18.

### Example 33: In Vitro Activity Test of Wild Type IFNα and Poly(amino acid)-IFNα Conjugates

In the example, the retention of in vitro anti-tumor and antiviral activities of various poly(amino acid)-IFNα was respectively determined from two aspects, i.e., growth inhibition of Daudi cells, and induced activities of A549 cells against encephalomyocarditis virus (EMCV).

In vitro anti-tumor activities of various poly(amino acid)-IFNα were detected with IFNα-sensitive Daudi cells of human B lymphoma cell line as the experimental cell line. Specific experimental steps included: cells were spread in a 96-well plate at a density of 5000 cells/well. A protein and a conjugate of the protein and poly(amino acid) were diluted to proper gradients, and then added to the 96-well plate spread with cells. After incubation for 72 h, the cell viability was detected by Celltiter Blue^{®} Cell Viability Assay (Promega), and relevant data were processed by Graphpad Prism to obtain half maximal inhibitory concentration (IC₅₀) of various samples. The experiment shows that anti-tumor activities of poly(amino acid)-IFNα conjugates having different topological structures are greatly different, and the specific data are shown in Table 2. In Table 2, conjugation of a poly(amino acid) having a secondary structure with an IFNα has least effect on its activity, and is superior to a clinically applied IFNα-polyethylene glycol conjugate (PEG-INTRON). The cyclic conjugates take the second place. Therefore, it can be seen that by adjusting the secondary structure or topological structure of the IFNα-poly(amino acid) conjugate, its thermal stability and enzymatic resistance are enhanced while retaining its activities to the greatest extent.

In the experiment, in vitro antiviral activities were determined by detecting activities of the A549 cells stimulated by a poly(amino acid)-IFNα conjugate against encephalomyocarditis virus (EMCV). Specific determination steps included: A549 cells were spread in a black non-transparent 96-well plate at a density of 8000-10000 cells/well. 24 h later, after the cells were fully adherent, a poly(amino acid)-interferon conjugate diluted in a DMEM containing 10% FBS (10 µg/mL, diluted 3X, totally 14 concentration points) was added at a density of 100 µL/well. After co-incubation with cells for 24 h, the protein solution was absorbed, and a certain concentration of EMCV solution was added (diluted to 2% FBS, in a DMEM, the amount of added virus must ensure that all cells without addition of protein must be pathologically changed within 48 h). The wells without addition of a protein and a virus were used as control of 100% cell survival, and the wells with the addition of a virus but without addition of protein were used as control of 0% cell survival. After 48 h, the cell viability was detected by CellTiter glo^{®} Luminescent Cell Viability Assay (Promega) to determine the antiviral activities of various conjugates.

**Table 2**

| | Wild type interferon | PAAP2 (n=20, m=3)-IFNα conjugate | Cyclic conjugate of PAAP2 (n=20, m=3)-IFNα | PAAP1 (n=100, L-type)-IFN α conjugate | PAAP1 (n=100, D,L-type)-IFN α conjugate | PEG-INTRON |
|---|---|---|---|---|---|---|
| IC₅₀ (pg/mL) | 8.5 | 82 | 97 | 36 | 160 | 66 |
| Relative anti-tumor activity | 100% | 26% | 28% | 31% | 12% | - |
| t_{1/2}(h) | 0.4 | 6.5 | 7.5 | 9.6 | 7.8 | 9.8 |

### Example 34: Determination of Plasma Concentration of wt-IFNα and Poly(amino acid)-IFNα Conjugates

In the example, SD female rats were used as experimental subjects of living animals. The SD rats were subjected to jugular vein intubation, revived for 48 hours, and injected with the samples to be tested through the vein intubation at a dose of 150 µg/Kg. Each drug was experimented on 3 rats, and blood samples were taken at 1 min, 15 min, 30 min, 1h, 3 h, 6 h, 9 h, 12 h, 24 h, 48 h, and 72 h, respectively. After storage on ice for half an hour, the blood samples were centrifuged at 4000 g, and the supernatant was kept at -80 °C.

The IFNα content in serum was detected by ELISA. The concentration change curves of the protein samples in blood are shown in Fig. 19, and the plasma concentration half life (t_{1/2}) is shown in Table 2. The protein-poly(amino acid) conjugate exhibits much longer plasma concentration half-life than WT-IFNα2b. The conjugate ligated with a L-poly(amino acid) (t_{1/2}=9.6 h) shows an effect that is almost consistent with clinically applied PEG-INTRON (t_{1/2}=9.8 h), and also has stronger effect than the conjugate ligated with D,L-poly(amino acid) (t_{1/2}=7.8 h), proving that the secondary structure of a poly(amino acid) will adjust the entire secondary structure of the protein-poly(amino acid) conjugate, thereby improving its biological properties and functions. The blood circulation time of the poly(amino acid)-interferon α cyclic conjugate (t_{1/2}=7.5 h) is longer than the blood circulation time (t_{1/2}=6.5 h) of the poly(amino acid)-interferon α conjugate before cyclization, suggesting that the topological structure of the poly(amino acid)-interferon α conjugate also has a great influence on the blood circulation.

### Example 35: In Vivo Antitumor Activities of wt-IFNα, Poly(amino acid) P2 (n=20, m=3)-IFNα Conjugate and Cyclic conjugate of Poly(amino acid) P2 (n=20, m=3)-IFNα

In the example, a tumor model was established in six-week-old Balb/c female nude mice with a human ovarian cancer cell (OVCAR-3) as the researched tumor cell line. Cells were suspended in 1640/matrigel (1:1 mixing ratio) at a density of 10⁷/200 µL, and subcutaneously injected into right front chests of the nude mice at a dose of 200 µL/nude mouse. Three weeks later, the tumor size was about 30 mm³. The mice were randomly divided into four groups with 5 mice in each group, and separately injected with a PBS, a wt-IFNα, a poly(amino acid) P2 (n=20, m=3)-IFNα conjugate and a cyclic conjugate of poly(amino acid) P2 (n=20, m=3)-IFNα at a dose of 20 µg IFNα/nude mouse, and at an injection volume of 100 µL, once a week. The tumor size and body weight of nude mice were monitored once every 3 days.

### Example 36: Expression and Purification of Cys-Human Epidermal Growth Factor Receptor 2 Binding Fragment (Cys-Her2 Fab Antibody)

A plasmid carrying Cys-Her2 Fab (cysteine was inserted into the N-terminus of the light chain) antibody was chemically transformed into competent cells of TOP10 E. coli, revived from 10 mL of LB medium at 250 rpm at 37 °C overnight, inoculated into 1L of LTB medium at a ratio of 1:100, and shaken at 250 rpm at 37 °C until OD₆₀₀=0.8. The protein expression was induced by adding arabinose at a final concentration of 0.2% at 200 rpm at 30 °C for 16-20 h. Upon completion of the expression, bacteria were collected by centrifugation at 6500 rpm for 30 min. 150 mL of a lysate (30 mM Tris, 1 mM EDTA, pH 8.0, 20% sucrose) was added, and gently stirred at room temperature for half an hour to uniformly disperse the bacteria in the lysate. Lysozyme with a final concentration of 0.2 mg/mL and 2.5 U/L benzonase nuclease were added, and shaken at 250 rpm at 37 °C for 30 min. The lysate was successively centrifuged at 6500 rpm for 30 min and centrifuged at 12000 g for 30 min, and the supernatant was retained each time. The supernatant was filtered through a 0.22µm filter membrane, and purified through a protein G resin. After sample injection, it was washed with a sodium acetate buffer (pH 4.5) of 4-5 column volumes, and the antibody was eluted with a 50 mM glycine solution (5-8 column volumes) at pH 2.8 to obtain Cys-Her2 Fab antibody. The molecular weight of the protein was identified by UPLC-MS after reduction with dithiothreitol. The theoretical molecular weight of the heavy chain of the antibody is 24305, and that of the light chain is 23674. The observed molecular weight of the heavy chain of the antibody is 24310, and that of the light chain is 23682. That is, the molecular weights comply with the theoretical calculated values (error falls within the allowable range). Therefore, the obtained antibody can be identified as the target antibody.

### Example 37: Native Chemical Ligation between Poly(amino acid) P1 (n=7) and Cys-Her2 Fab Antibody

A Cys-Her2 Fab (5 mg/mL, 50 µL, 1.0 equiv) was mixed with a poly(amino acid) P1 (n=7) (30 equiv), and reacted at room temperature for 10-12 h. The mixture was purified by FPLC to obtain a poly(amino acid) P1 (n=7)-Her2 Fab antibody conjugate, the purity and the molecular weight of which were characterized through SDS-PAGE. After reduction with dithiothreitol, the heavy chain and light chain of the antibody were isolated to obtain bands with about halved molecular weights, as shown in Fig. 21. The purified product migrates to high molecular weight relative to the antibody before reaction, and has a homogeneous band before reduction, indicating that the poly(amino acid) is successfully grafted, and the product has a high purity. The product yield was 26%.

In view of the above, the protein-polymer conjugate according to the disclosure has the features of simple preparation process and high controllability, thus greatly reducing the cost of modifying proteins, enabling in vivo properties of the proteins to be more stable, further improving the blood circulation time, and effectively achieving biological and pharmacological effects of the proteins.

## Claims

1. A protein-poly(amino acid) conjugate, having a structure represented by general formula 1: 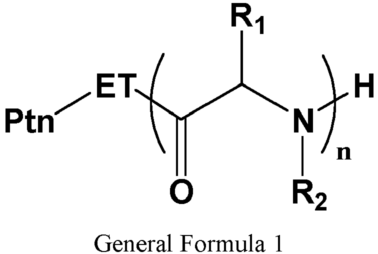 wherein
Ptn represents a protein;
ET is 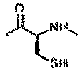 when it is connected with the N-terminus of the Ptn, and is 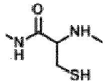 when it is connected with a site other than the N-terminus of the Ptn;
R₁ independently represents, at each occurrence, a side chain of a non-natural amino acid;
R₂ independently represents, at each occurrence, hydrogen or C₁-C₁₀ alkyl, C₂-C₁₀ alkenyl, C₂-C₁₀ alkynyl; and
n is an integer selected from 1 to 200.

2. The protein-poly(amino acid) conjugate according to claim 1, wherein the Ptn is a protein comprising a polypeptide hormone, a monoclonal antibody, a genetically engineered antibody, an interferon, an interleukin, a colony stimulating factor or a recombinant vaccine, and preferably, the Ptn is interferon α2b; optionally R₁ represents, at each occurrence, a side chain of tyrosine, serine, threonine, cysteine, aspartic acid and/or glutamic acid modified with oligo(ethylene glycol), phosphate, propenyloxybenzyl ester or allyl triethylene glycol; optionally wherein R₂ represents, at each occurrence, hydrogen or C₁-C₁₀ alkyl.

3. A protein-poly(amino acid) conjugate, having a structure represented by general formula 2: 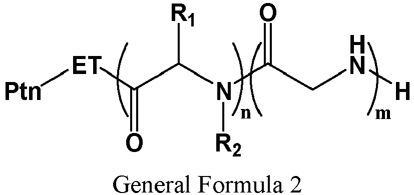 wherein
Ptn, ET, R₁ and R₂ are as defined in claim 1 or 2;
n is an integer selected from 1 to 200; and
m is an integer selected from 1 to 30.

4. A protein-poly(amino acid) cyclic conjugate, having a structure represented by general formula 3: 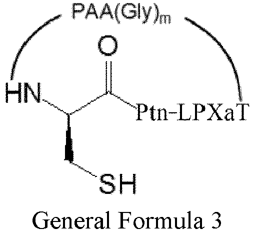 wherein
Ptn represents a protein having a cysteine residue at the N-terminus and a LPXaT sequence at the C-terminus, in which Xa represents one or more amino acids;
PAA(Gly)ₘ is a structure represented by general formula 4: 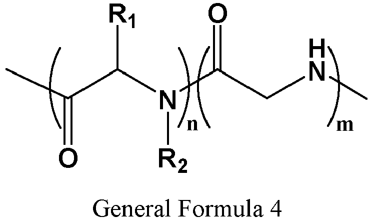
wherein
R₁ independently represents, at each occurrence, a side chain of a non-natural amino acid;
R₂ independently represents, at each occurrence, hydrogen or C₁-C₁₀ alkyl, C₂-C₁₀ alkenyl, C₂-C₁₀ alkynyl;
n is an integer selected from 10 to 200; and
m is an integer selected from 1 to 30.

5. The protein-poly(amino acid) cyclic conjugate according to claim 4, wherein the Ptn is a protein comprising a polypeptide hormone, a monoclonal antibody, a genetically engineered antibody, an interferon, an interleukin, a colony stimulating factor or a recombinant vaccine, and preferably, the Ptn is interferon α2b; optionally R₁ represents, at each occurrence, a side chain of tyrosine, serine, threonine, cysteine, aspartic acid and/or glutamic acid modified with oligo(ethylene glycol), phosphate, propenyloxybenzyl ester or allyl triethylene glycol; optionally wherein R₂ represents, at each occurrence, hydrogen or C₁-C₁₀ alkyl.

6. A poly(amino acid) compound, having a structure represented by general formula 5: 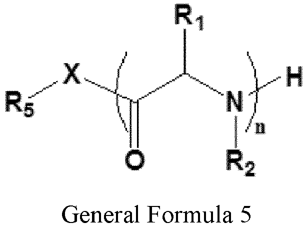 wherein
X represents sulphur or selenium;
R₁ independently represents, at each occurrence, a side chain of a non-natural amino acid;
R₂ independently represents, at each occurrence, hydrogen or C₁-C₁₀ alkyl, C₂-C₁₀ alkenyl, C₂-C₁₀ alkynyl;
R₅ represents substituted or unsubstituted C₁-C₃₀ alkyl, substituted or unsubstituted C₂-C₃₀ alkenyl, substituted or unsubstituted C₂-C₃₀ alkynyl, substituted or unsubstituted C₃-C₃₀ cycloalkyl, substituted or unsubstituted C₃-C₃₀ cycloalkenyl, substituted or unsubstituted C₁-C₃₀ heteroalkyl, substituted or unsubstituted C₂-C₃₀ heteroalkenyl, substituted or unsubstituted C₂-C₃₀ heteroalkynyl, substituted or unsubstituted C₁-C₃₀ heterocycloalkyl, substituted or unsubstituted C₂-C₃₀ heterocycloalkenyl, substituted or unsubstituted C₆-C₃₀ aryl, or substituted or unsubstituted C₅-C₃₀ heteroaryl; and
n is an integer selected from 1 to 200.

7. The poly(amino acid) compound according to claim 6, wherein R₁ represents, at each occurrence, a side chain of tyrosine, serine, threonine, cysteine, aspartic acid and/or glutamic acid modified with oligo(ethylene glycol), phosphate, propenyloxybenzyl ester or allyl triethylene glycol; optionally wherein R₂ represents, at each occurrence, hydrogen or C₁-C₁₀ alkyl; optionally wherein R₅ represents substituted or unsubstituted C₁-C₁₀ alkyl, substituted or unsubstituted C₂-C₁₀ alkenyl, substituted or unsubstituted C₂-C₁₀ alkynyl, substituted or unsubstituted C₃-C₁₀ cycloalkyl, substituted or unsubstituted C₃-C₁₀ cycloalkenyl, substituted or unsubstituted C₁-C₁₀ heteroalkyl, substituted or unsubstituted C₂-C₁₀ heteroalkenyl, substituted or unsubstituted C₁-C₁₀ heterocycloalkyl, substituted or unsubstituted C₂-C₁₀ heterocycloalkenyl, substituted or unsubstituted C₆-C₁₈ aryl, substituted or unsubstituted C₅-C₁₈ heteroaryl, wherein a substituent for substituting R₅ is selected from the group consisting of C₁₋₃ haloalkyl, hydroxy, amino, mercapto, carbonyl, carboxy, sulfo, carboxylate, sulfonate, ester group, amide and/or halogen; and preferably, R₅ represents substituted or unsubstituted C₁-C₁₀ alkyl, substituted or unsubstituted C₃-C₁₀ cycloalkyl, substituted or unsubstituted C₁-C₁₀ heteroalkyl, substituted or unsubstituted C₁-C₁₀ heterocycloalkyl, substituted or unsubstituted C₆-C₁₈ aryl, or substituted or unsubstituted C₅-C₁₈ heteroaryl.

8. The poly(amino acid) compound according to claims 6 or 7, wherein R₅ is selected from the following structures:

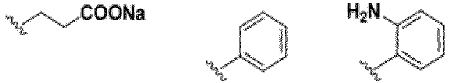

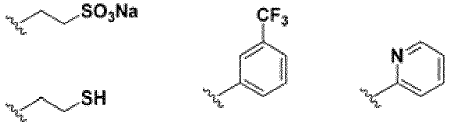

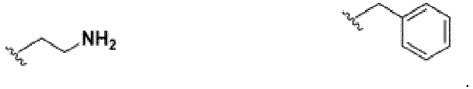

9. A poly(amino acid) compound, having a structure represented by general formula 6: 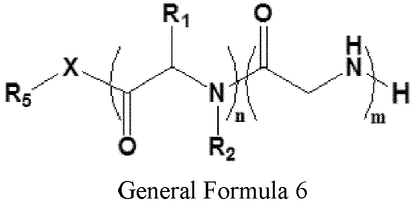 wherein
X represents sulphur or selenium;
R₁ independently represents, at each occurrence, a side chain of a non-natural amino acid;
R₂ independently represents, at each occurrence, hydrogen or C₁-C₁₀ alkyl, C₂-C₁₀ alkenyl, C₂-C₁₀ alkynyl;
R₅ represents substituted or unsubstituted C₁-C₃₀ alkyl, substituted or unsubstituted C₂-C₃₀ alkenyl, substituted or unsubstituted C₂-C₃₀ alkynyl, substituted or unsubstituted C₃-C₃₀ cycloalkyl, substituted or unsubstituted C₃-C₃₀ cycloalkenyl, substituted or unsubstituted C₁-C₃₀ heteroalkyl, substituted or unsubstituted C₂-C₃₀ heteroalkenyl, substituted or unsubstituted C₂-C₃₀ heteroalkynyl, substituted or unsubstituted C₁-C₃₀ heterocycloalkyl, substituted or unsubstituted C₂-C₃₀ heterocycloalkenyl, substituted or unsubstituted C₆-C₃₀ aryl, or substituted or unsubstituted C₅-C₃₀ heteroaryl;
n is an integer selected from 10 to 200; and
m is an integer selected from 1 to 30.

10. A method for preparing a protein-poly(amino acid) conjugate according to any one of claims 1 to 3, comprising: (1) initiating polymerization of a N-carboxyanhydride by an initiator R₅XY to obtain a poly(amino acid) having a carbon structure; and (2) mixing the poly(amino acid) with a protein containing a 1,2-mercaptoethylamine structure to obtain the site-specific protein-poly(amino acid) conjugate through native chemical ligation,
wherein
X represents sulphur or selenium;
Y represents hydrogen or trialkylsilyl, wherein the alkyl moiety in the trialkylsilyl is C₁-C₁₀ alkyl; and
R₅ represents substituted or unsubstituted C₁-C₃₀ alkyl, substituted or unsubstituted C₂-C₃₀ alkenyl, substituted or unsubstituted C₂-C₃₀ alkynyl, substituted or unsubstituted C₃-C₃₀ cycloalkyl, substituted or unsubstituted C₃-C₃₀ cycloalkenyl, substituted or unsubstituted C₁-C₃₀ heteroalkyl, substituted or unsubstituted C₂-C₃₀ heteroalkenyl, substituted or unsubstituted C₂-C₃₀ heteroalkynyl, substituted or unsubstituted C₁-C₃₀ heterocycloalkyl, substituted or unsubstituted C₂-C₃₀ heterocycloalkenyl, substituted or unsubstituted C₆-C₃₀ aryl, or substituted or unsubstituted C₅-C₃₀ heteroaryl.

11. The method according to claim 10, wherein in the step (1), the polymerization of the N-carboxyanhydride is initiated by the initiator R₅XY to obtain a poly(amino acid) PAA-XR₅ containing a carbon structure: 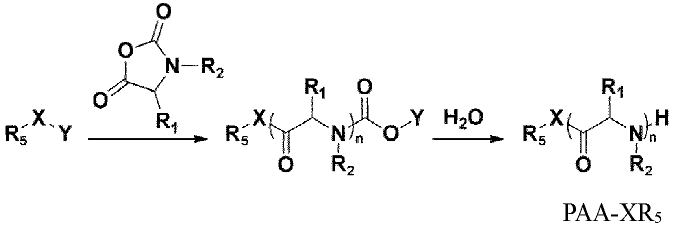 wherein
R₁ independently represents, at each occurrence, a side chain of a non-natural amino acid;
R₂ independently represents, at each occurrence, hydrogen or C₁-C₁₀ alkyl, C₂-C₁₀ alkenyl, C₂-C₁₀ alkynyl; and
n is an integer selected from 1 to 200.

12. The method according to claim 11, wherein R₂ is hydrogen.

13. The method according to claim 11 or 12, wherein the R₅XY is trimethylsilyl benzenethiol or trimethylsilyl benzeneselenol, optionally wherein the step (1) is carried out in an aprotic solvent at room temperature, optionally wherein the poly(amino acid) obtained in the step (1) is subjected to a post-modification, and the post-modification comprises mixing the poly(amino acid) with a modifier and exposing the mixture to an ultraviolet lamp, and the modifier is mercaptoethylamine hydrochloride or mercaptopropionic acid.

14. The method according to claim 12, wherein in the step (2), the poly(amino acid) obtained in the step (1) is mixed with the protein containing a 1,2-mercaptoethylamine structure to obtain the site-specific protein-poly(amino acid) conjugate through native chemical ligation: 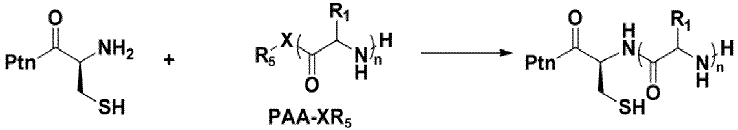 wherein the Ptn represents a protein.

15. The method according to claim 11, wherein in the step (2), the poly(amino acid) obtained in the step (1) is mixed with the protein containing a 1,2-mercaptoethylamine structure to obtain the site-specific protein-poly(amino acid) conjugate through native chemical ligation: 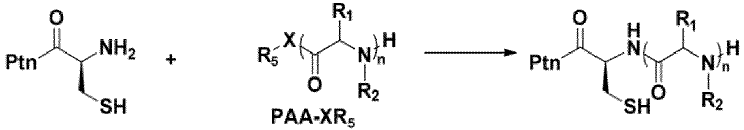 wherein the Ptn represents a protein.

16. A method for preparing a protein-poly(amino acid) cyclic conjugate according to claim 4 or 5, comprising: (1) initiating polymerization of a N-carboxyanhydride and a glycine N-carboxyanhydride by an initiator R₅XY to obtain a block poly(amino acid) having a phenylthioester structure at the C-terminus and a block polyglycine structure at the N-terminus; and (2) mixing the block poly(amino acid) with a protein having a cysteine at the N-terminus and a LPXaTG sequence at the C-terminus to successively conduct native chemical ligation and sortase mediated reaction to achieve cyclization of the protein, wherein the LPXaTG sequence is a recognition site of the sortase,
wherein
X represents sulphur;
Y represents hydrogen or trialkylsilyl, wherein the alkyl moiety in the trialkylsilyl is C₁-C₁₀ alkyl; and
R₅ represents phenyl group.

17. The method according to claim 16, wherein in the step (1), a polymerization of the N-carboxyanhydride and another N-carboxyanhydride is initiated by the initiator R₅XY to obtain a block poly(amino acid) having a phenylthioester structure at the C-terminus and a block polyglycine structure at the N-terminus: 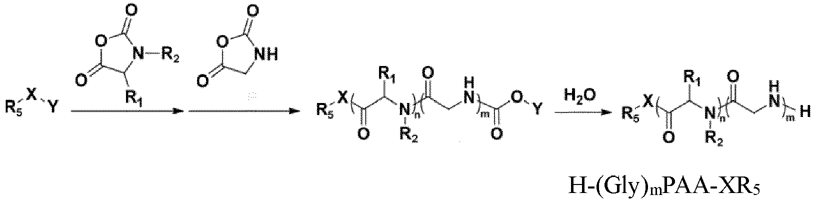 wherein
R₁ independently represents, at each occurrence, a side chain of a non-natural amino acid;
R₂ independently represents, at each occurrence, hydrogen or C₁-C₁₀ alkyl, C₂-C₁₀ alkenyl, C₂-C₁₀ alkynyl;
n is an integer selected from 10 to 200; and
m is an integer selected from 1 to 30.

18. The method according to claim 17, wherein R₂ is hydrogen.

19. The method according to claim 17 or 18, wherein the R₅XY is benzenethiol; optionally wherein the step (1) is carried out in an aprotic solvent at room temperature; optionally wherein the poly(amino acid) obtained in the step (1) is subjected to a post-modification, and the post-modification comprises mixing the poly(amino acid) with a modifier and exposing the mixture to an ultraviolet lamp, and the modifier is mercaptoethylamine hydrochloride or mercaptopropionic acid.

20. The method according to claim 17 or 18, wherein in the step (2), the block poly(amino acid) obtained in the step (1) is mixed with the protein having a cysteine at the N-terminus and a LPXaTG sequence at the C-terminus, the block poly(amino acid) is ligated with the cysteine at the N-terminus of the protein, and then cyclization is achieved in the presence of sortase: 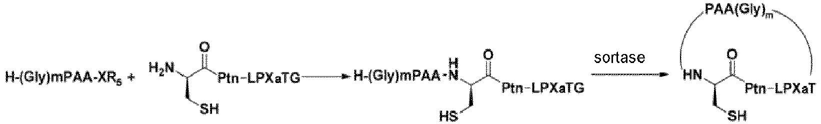 wherein the LPXaTG sequence is a recognition site of the sortase.

## Patentansprüche

1. Protein-Poly(aminosäure)-Konjugat, das eine Struktur aufweist, die durch die allgemeine Formel 1 dargestellt ist: 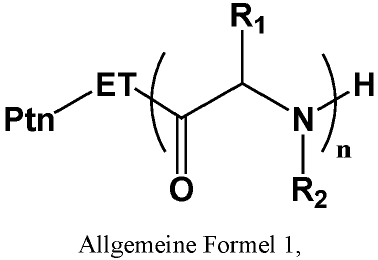 wobei
Ptn ein Protein darstellt;
ET 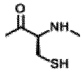 ist, wenn es mit dem N-Terminus des Ptn verbunden ist, und 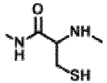 ist, wenn es mit einer Stelle außer dem N-Terminus des Ptn verbunden ist;
R₁ unabhängig bei jedem Auftreten eine Seitenkette einer nichtnatürlichen Aminosäure darstellt;
R₂ unabhängig bei jedem Auftreten Wasserstoff oder C₁-C₁₀-Alkyl, C₂-C₁₀-Alkenyl, C₂-C₁₀-Alkinyl darstellt; und
n eine ganze Zahl ist, die aus 1 bis 200 ausgewählt ist.

2. Protein-Poly(aminosäure)-Konjugat nach Anspruch 1, wobei das Ptn ein Protein ist, das Folgendes umfasst: ein Polypeptidhormon, einen monoklonalen Antikörper, einen gentechnisch hergestellten Antikörper, ein Interferon, ein Interleukin, einen koloniestimulierenden Faktor oder einen rekombinanten Impfstoff, und bevorzugt das Ptn Interferon a2b ist; optional R₁ bei jedem Auftreten Folgendes darstellt: eine Seitenkette von Tyrosin, Serin, Threonin, Cystein, Asparaginsäure und/oder Glutaminsäure, das/die mit Oligo(ethylenglycol), Phosphat, Propenyloxybenzylester oder Allyltriethylenglycol modifiziert ist; optional wobei R₂ bei jedem Auftreten Wasserstoff oder C₁-C₁₀-Alkyl darstellt.

3. Protein-Poly(aminosäure)-Konjugat, das eine Struktur aufweist, die durch die allgemeine Formel 2 dargestellt ist: 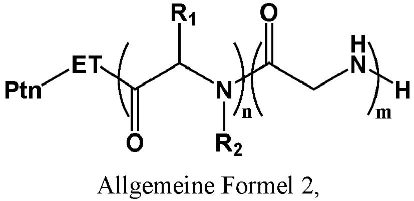 wobei
Ptn, ET, R₁ und R₂ wie in Anspruch 1 oder 2 definiert sind;
n eine ganze Zahl ist, die aus 1 bis 200 ausgewählt ist; und
m eine ganze Zahl ist, die aus 1 bis 30 ausgewählt ist.

4. Cyclisches Protein-Poly(aminosäure)-Konjugat, das eine Struktur aufweist, die durch die allgemeine Formel 3 dargestellt ist: 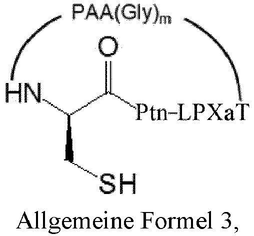 wobei
Ptn ein Protein darstellt, das einen Cysteinrest an dem N-Terminus und eine LPXaT-Sequenz an dem C-Terminus aufweist, in der Xa eine oder mehrere Aminosäuren darstellt;
PAA(Gly)ₘ eine Struktur ist, die durch die allgemeine Formel 4 dargestellt ist: 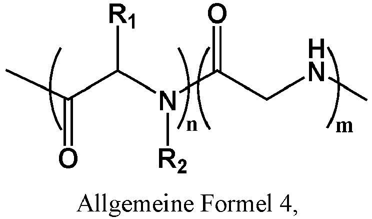
wobei
R₁ unabhängig bei jedem Auftreten eine Seitenkette einer nichtnatürlichen Aminosäure darstellt;
R₂ unabhängig bei jedem Auftreten Wasserstoff oder C₁-C₁₀-Alkyl, C₂-C₁₀-Alkenyl, C₂-C₁₀-Alkinyl darstellt;
n eine ganze Zahl ist, die aus 10 bis 200 ausgewählt ist; und
m eine ganze Zahl ist, die aus 1 bis 30 ausgewählt ist.

5. Cyclisches Protein-Poly(aminosäure)-Konjugat nach Anspruch 4, wobei das Ptn ein Protein ist, das Folgendes umfasst: ein Polypeptidhormon, einen monoklonalen Antikörper, einen gentechnisch hergestellten Antikörper, ein Interferon, ein Interleukin, einen koloniestimulierenden Faktor oder einen rekombinanten Impfstoff, und bevorzugt das Ptn Interferon a2b ist; optional R₁ bei jedem Auftreten Folgendes darstellt: eine Seitenkette von Tyrosin, Serin, Threonin, Cystein, Asparaginsäure und/oder Glutaminsäure, das/die mit Oligo(ethylenglycol), Phosphat, Propenyloxybenzylester oder Allyltriethylenglycol modifiziert ist; optional wobei R₂ bei jedem Auftreten Wasserstoff oder C₁-C₁₀-Alkyl darstellt.

6. Poly(aminosäure)-Verbindung, die eine Struktur aufweist, die durch die allgemeine Formel 5 dargestellt ist: 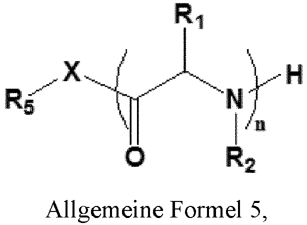 wobei
X Schwefel oder Selen darstellt;
R₁ unabhängig bei jedem Auftreten eine Seitenkette einer nichtnatürlichen Aminosäure darstellt;
R₂ unabhängig bei jedem Auftreten Wasserstoff oder C₁-C₁₀-Alkyl, C₂-C₁₀-Alkenyl, C₂-C₁₀-Alkinyl darstellt;
R₅ Folgendes darstellt: substituiertes oder unsubstituiertes C₁-C₃₀-Alkyl, substituiertes oder unsubstituiertes C₂-C₃₀-Alkenyl, substituiertes oder unsubstituiertes C₂-C₃₀-Alkinyl, substituiertes oder unsubstituiertes C₃-C₃₀-Cycloalkyl, substituiertes oder unsubstituiertes C₃-C₃₀-Cycloalkenyl, substituiertes oder unsubstituiertes C₁-C₃₀-Heteroalkyl, substituiertes oder unsubstituiertes C₂-C₃₀-Heteroalkenyl, substituiertes oder unsubstituiertes C₂-C₃₀-Heteroalkinyl, substituiertes oder unsubstituiertes C₁-C₃₀-Heterocycloalkyl, substituiertes oder unsubstituiertes C₂-C₃₀-Heterocycloalkenyl, substituiertes oder unsubstituiertes C₆-C₃₀-Aryl oder substituiertes oder unsubstituiertes C₅-C₃₀-Heteroaryl; und
n eine ganze Zahl ist, die aus 1 bis 200 ausgewählt ist.

7. Poly(aminosäure)-Verbindung nach Anspruch 6, wobei R₁ bei jedem Auftreten Folgendes darstellt: eine Seitenkette von Tyrosin, Serin, Threonin, Cystein, Asparaginsäure und/oder Glutaminsäure, das/die mit Oligo(ethylenglycol), Phosphat, Propenyloxybenzylester oder Allyltriethylenglycol modifiziert ist; optional wobei R₂ bei jedem Auftreten Wasserstoff oder C₁-C₁₀-Alkyl darstellt; optional wobei R₅ Folgendes darstellt: substituiertes oder unsubstituiertes C₁-C₁₀-Alkyl, substituiertes oder unsubstituiertes C₂-C₁₀-Alkenyl, substituiertes oder unsubstituiertes C₂-C₁₀-Alkinyl, substituiertes oder unsubstituiertes C₃-C₁₀-Cycloalkyl, substituiertes oder unsubstituiertes C₃-C₁₀-Cycloalkenyl, substituiertes oder unsubstituiertes C₁-C₁₀-Heteroalkyl, substituiertes oder unsubstituiertes C₂-C₁₀-Heteroalkenyl, substituiertes oder unsubstituiertes C₁-C₁₀-Heterocycloalkyl, substituiertes oder unsubstituiertes C₂-C₁₀-Heterocycloalkenyl, substituiertes oder unsubstituiertes C₆-C₁₈-Aryl, substituiertes oder unsubstituiertes Cs-Cis-Heteroaryl, wobei ein Substituent für die Substitution von R₅ aus der Gruppe ausgewählt ist, die aus Folgenden besteht: C₁₋₃-Halogenalkyl, Hydroxy, Amino, Mercapto, Carbonyl, Carboxy, Sulfo, Carboxylat, Sulfonat, Estergruppe, Amid und/oder Halogen; und bevorzugt R₅ Folgendes darstellt: substituiertes oder unsubstituiertes C₁-C₁₀-Alkyl, substituiertes oder unsubstituiertes C₃-C₁₀-Cycloalkyl, substituiertes oder unsubstituiertes C₁-C₁₀-Heteroalkyl, substituiertes oder unsubstituiertes C₁-C₁₀-Heterocycloalkyl, substituiertes oder unsubstituiertes C₆-Cis-Aryl oder substituiertes oder unsubstituiertes C₅-C₁₈-Heteroaryl.

8. Poly(aminosäure)-Verbindung nach den Ansprüchen 6 oder 7, wobei R₅ aus den folgenden Strukturen ausgewählt ist:

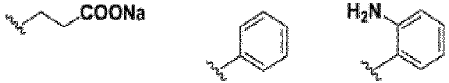

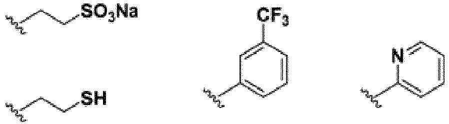

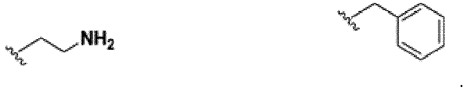

9. Poly(aminosäure)-Verbindung, die eine Struktur aufweist, die durch die allgemeine Formel 6 dargestellt ist: 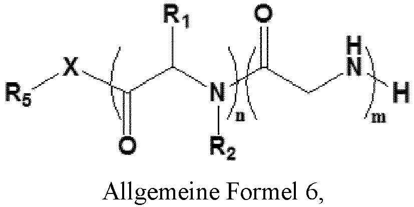 wobei
X Schwefel oder Selen darstellt;
R₁ unabhängig bei jedem Auftreten eine Seitenkette einer nichtnatürlichen Aminosäure darstellt;
R₂ unabhängig bei jedem Auftreten Wasserstoff oder C₁-C₁₀-Alkyl, C₂-C₁₀-Alkenyl, C₂-C₁₀-Alkinyl darstellt;
R₅ Folgendes darstellt: substituiertes oder unsubstituiertes C₁-C₃₀-Alkyl, substituiertes oder unsubstituiertes C₂-C₃₀-Alkenyl, substituiertes oder unsubstituiertes C₂-C₃₀-Alkinyl, substituiertes oder unsubstituiertes C₃-C₃₀-Cycloalkyl, substituiertes oder unsubstituiertes C₃-C₃₀-Cycloalkenyl, substituiertes oder unsubstituiertes C₁-C₃₀-Heteroalkyl, substituiertes oder unsubstituiertes C₂-C₃₀-Heteroalkenyl, substituiertes oder unsubstituiertes C₂-C₃₀-Heteroalkinyl, substituiertes oder unsubstituiertes C₁-C₃₀-Heterocycloalkyl, substituiertes oder unsubstituiertes C₂-C₃₀-Heterocycloalkenyl, substituiertes oder unsubstituiertes C₆-C₃₀-Aryl oder substituiertes oder unsubstituiertes C₅-C₃₀-Heteroaryl;
n eine ganze Zahl ist, die aus 10 bis 200 ausgewählt ist; und
m eine ganze Zahl ist, die aus 1 bis 30 ausgewählt ist.

10. Verfahren für die Herstellung eines Protein-Poly(aminosäure)-Konjugats nach einem der Ansprüche 1 bis 3, das Folgendes umfasst: (1) Auslösen von Polymerisation eines N-Carboxyanhydrids durch einen Initiator R₅XY, um eine Poly(aminosäure) zu erhalten, die eine Kohlenstoffstruktur aufweist; und (2) Mischen der Poly(aminosäure) mit einem Protein, das eine 1,2-Mercaptoethylamin-Struktur enthält, um ein ortsspezifisches Protein-Poly(aminosäure)-Konjugat durch native chemische Ligation zu erhalten,
wobei
X Schwefel oder Selen darstellt;
Y Wasserstoff oder Trialkylsilyl darstellt, wobei die Alkyleinheit in dem Trialkylsilyl C₁-C₁₀-Alkyl ist; und
R₅ Folgendes darstellt: substituiertes oder unsubstituiertes C₁-C₃₀-Alkyl, substituiertes oder unsubstituiertes C₂-C₃₀-Alkenyl, substituiertes oder unsubstituiertes C₂-C₃₀-Alkinyl, substituiertes oder unsubstituiertes C₃-C₃₀-Cycloalkyl, substituiertes oder unsubstituiertes C₃-C₃₀-Cycloalkenyl, substituiertes oder unsubstituiertes C₁-C₃₀-Heteroalkyl, substituiertes oder unsubstituiertes C₂-C₃₀-Heteroalkenyl, substituiertes oder unsubstituiertes C₂-C₃₀-Heteroalkinyl, substituiertes oder unsubstituiertes C₁-C₃₀-Heterocycloalkyl, substituiertes oder unsubstituiertes C₂-C₃₀-Heterocycloalkenyl, substituiertes oder unsubstituiertes C₆-C₃₀-Aryl oder substituiertes oder unsubstituiertes C₅-C₃₀-Heteroaryl.

11. Verfahren nach Anspruch 10, wobei in dem Schritt (1) die Polymerisation des N-Carboxyanhydrids durch den Initiator R₅XY ausgelöst wird, um eine Poly(aminosäure) PAA-XR₅ zu erhalten, die eine Kohlenstoffstruktur enthält: 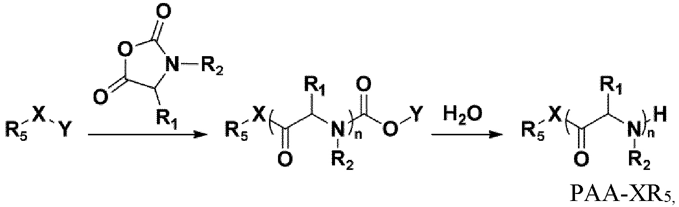 wobei
R₁ unabhängig bei jedem Auftreten eine Seitenkette einer nichtnatürlichen Aminosäure darstellt;
R₂ unabhängig bei jedem Auftreten Wasserstoff oder C₁-C₁₀-Alkyl, C₂-C₁₀-Alkenyl, C₂-C₁₀-Alkinyl darstellt; und
n eine ganze Zahl ist, die aus 1 bis 200 ausgewählt ist.

12. Verfahren nach Anspruch 11, wobei R₂ Wasserstoff ist.

13. Verfahren nach Anspruch 11 oder 12, wobei der R₅XY Trimethylsilylbenzolthiol oder Trimethylsilylbenzolselenol ist, optional wobei der Schritt (1) in einem aprotischen Lösungsmittel bei Raumtemperatur durchgeführt wird, optional wobei die Poly(aminosäure), die im Schritt (1) erhalten wird, einer Postmodifikation unterzogen wird und die Postmodifikation das Mischen der Poly(aminosäure) mit einem Modifikator und Aussetzen der Mischung gegenüber einer Ultraviolett-Lampe umfasst und der Modifikator Mercaptoethylaminhydrochlorid oder Mercaptopropionsäure ist.

14. Verfahren nach Anspruch 12, wobei in dem Schritt (2) die Poly(aminosäure), die im Schritt (1) erhalten wird, mit dem Protein, das eine 1,2-Mercaptoethylamin-Struktur enthält, gemischt wird, um das ortsspezifisches Protein-Poly(aminosäure)-Konjugat durch native chemische Ligation zu erhalten: 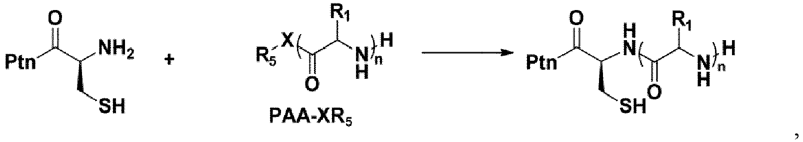 wobei das Ptn ein Protein darstellt.

15. Verfahren nach Anspruch 11, wobei in dem Schritt (2) die Poly(aminosäure), die im Schritt (1) erhalten wird, mit dem Protein, das eine 1,2-Mercaptoethylamin-Struktur enthält, gemischt wird, um das ortsspezifisches Protein-Poly(aminosäure)-Konjugat durch native chemische Ligation zu erhalten: 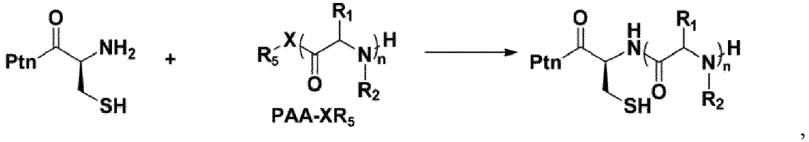 wobei das Ptn ein Protein darstellt.

16. Verfahren für die Herstellung eines cyclischen Protein-Poly(aminosäure)-Konjugats nach Anspruch 4 oder 5, das Folgendes umfasst: (1) Auslösen von Polymerisation eines N-Carboxyanhydrids und eines Glycin-N-carboxyanhydrids durch einen Initiator RsXY, um eine Block-Poly(aminosäure) zu erhalten, die eine Phenylthioester-Struktur an dem C-Terminus und eine Block-Polyglycin-Struktur an dem N-Terminus aufweist; und (2) Mischen der Block-Poly(aminosäure) mit einem Protein, das ein Cystein an dem N-Terminus und eine LPXaTG-Sequenz an dem C-Terminus aufweist, um aufeinanderfolgend eine native chemische Ligation und Sortasevermittelte Reaktion durchzuführen, um die Cyclisierung des Proteins zu erreichen, wobei die LPXaTG-Sequenz eine Erkennungsstelle der Sortase ist,
wobei
X Schwefel darstellt;
Y Wasserstoff oder Trialkylsilyl darstellt, wobei die Alkyleinheit in dem Trialkylsilyl C₁-C₁₀-Alkyl ist; und
R₅ eine Phenylgruppe darstellt.

17. Verfahren nach Anspruch 16, wobei in dem Schritt (1) eine Polymerisation des N-Carboxyanhydrids und eines weiteren N-Carboxyanhydrids durch den Initiator R₅XY ausgelöst wird, um eine Block-Poly(aminosäure) zu erhalten, die eine Phenylthioester-Struktur an dem C-Terminus und eine Block-Polyglycin-Struktur an dem N-Terminus aufweist: 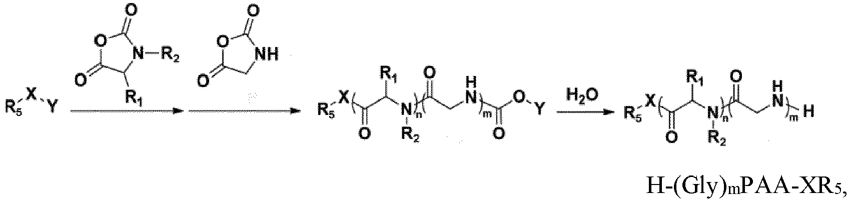 wobei
R₁ unabhängig bei jedem Auftreten eine Seitenkette einer nichtnatürlichen Aminosäure darstellt;
R₂ unabhängig bei jedem Auftreten Wasserstoff oder C₁-C₁₀-Alkyl, C₂-C₁₀-Alkenyl, C₂-C₁₀-Alkinyl darstellt;
n eine ganze Zahl ist, die aus 10 bis 200 ausgewählt ist; und
m eine ganze Zahl ist, die aus 1 bis 30 ausgewählt ist.

18. Verfahren nach Anspruch 17, wobei R₂ Wasserstoff ist.

19. Verfahren nach Anspruch 17 oder 18, wobei der R₅XY Benzolthiol ist; optional wobei der Schritt (1) in einem aprotischen Lösungsmittel bei Raumtemperatur durchgeführt wird; optional wobei die Poly(aminosäure), die im Schritt (1) erhalten wird, einer Postmodifikation unterzogen wird und die Postmodifikation das Mischen der Poly(aminosäure) mit einem Modifikator und Aussetzen der Mischung gegenüber einer Ultraviolett-Lampe umfasst und der Modifikator Mercaptoethylaminhydrochlorid oder Mercaptopropionsäure ist.

20. Verfahren nach Anspruch 17 oder 18, wobei in dem Schritt (2) die Block-Poly(aminosäure), die im Schritt (1) erhalten wird, mit dem Protein, das ein Cystein an dem N-Terminus und eine LPXaTG-Sequenz an dem C-Terminus aufweist, gemischt wird, die Block-Poly(aminosäure) mit dem Cystein an dem N-Terminus des Proteins ligiert wird und dann die Cyclisierung in Gegenwart von Sortase erreicht wird: 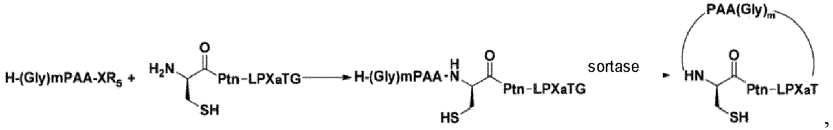 wobei die LPXaTG-Sequenz eine Erkennungsstelle der Sortase ist.

## Revendications

1. Conjugué protéine-polyaminoacide ayant une structure représentée par la Formule générale 1 : 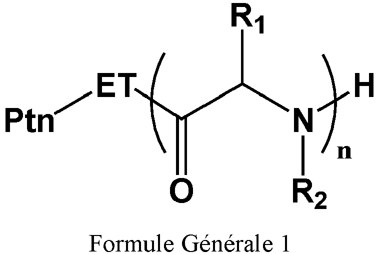 où
Ptn représente une protéine ;
ET représente 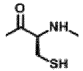 quand il est raccordé à l'extrémité N-terminale de
la Ptn et représente 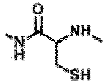 quand il est raccordé à un site autre que l'extrémité N-terminale de la Ptn ;
R₁ représente indépendamment, à chaque occurrence, une chaîne latérale d'un acide aminé artificiel ;
R₂ représente indépendamment, à chaque occurrence, un hydrogène ou un alkyle en C₁-C₁₀, alcényle en C₂-C₁₀, alcynyle en C₂-C₁₀ ; et
n est un nombre entier sélectionné dans la plage de 1 à 200.

2. Conjugué protéine-polyaminoacide selon la revendication 1, où la Ptn est une protéine comprenant une hormone polypeptidique, un anticorps monoclonal, un anticorps génétiquement modifié, un interféron, une interleukine, un facteur de stimulation de colonies ou un vaccin recombinant, et préférablement où la Ptn est l'interféron α2b ; éventuellement où R₁ représente, à chaque occurrence, une chaîne latérale de la tyrosine, de la sérine, de la thréonine, de la cystéine, de l'acide aspartique et/ou de l'acide glutamique modifiée par un oligo(éthylène glycol), un phosphate, un ester propényloxybenzylique ou un allyl-triéthylène glycol ; éventuellement où R₂ représente, à chaque occurrence, un hydrogène ou un alkyle en C₁-C₁₀.

3. Conjugué protéine-polyaminoacide ayant une structure représentée par la Formule générale 2 : 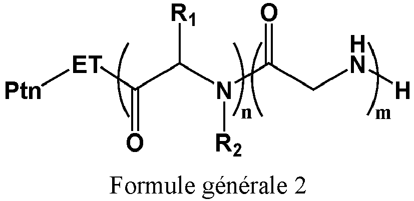 où
Ptn, ET, R₁ et R₂ sont tels que définis à la revendication 1 ou 2 ;
n est un nombre entier sélectionné dans la plage de 1 à 200 ; et
m est un nombre entier sélectionné dans la plage de 1 à 30.

4. Conjugué protéine-polyaminoacide cyclique ayant une structure représentée par la Formule générale 3 : 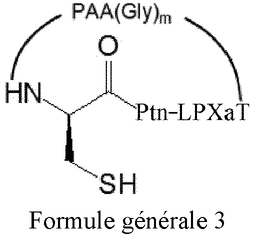 où
Ptn représente une protéine ayant un résidu cystéine à l'extrémité N-terminale et une séquence LPXaT dans laquelle Xa représente un ou plusieurs acides aminés à l'extrémité C-terminale ;
PAA(Gly)ₘ est une structure représentée par la Formule générale 4 : 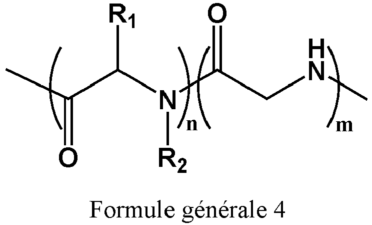
où
R₁ représente indépendamment, à chaque occurrence, une chaîne latérale d'un acide aminé artificiel ;
R₂ représente indépendamment, à chaque occurrence, un hydrogène ou un alkyle en C₁-C₁₀, alcényle en C₂-C₁₀, alcynyle en C₂-C₁₀ ;
n est un nombre entier sélectionné dans la plage de 10 à 200 ; et
m est un nombre entier sélectionné dans la plage de 1 à 30.

5. Conjugué protéine-polyaminoacide cyclique selon la revendication 4, où la Ptn est une protéine comprenant une hormone polypeptidique, un anticorps monoclonal, un anticorps génétiquement modifié, un interféron, une interleukine, un facteur de stimulation de colonies ou un vaccin recombinant, et préférablement où la Ptn est l'interféron a2b ; éventuellement où R₁ représente, à chaque occurrence, une chaîne latérale de la tyrosine, de la sérine, de la thréonine, de la cystéine, de l'acide aspartique et/ou de l'acide glutamique modifiée par un oligo(éthylène glycol), un phosphate, un ester propényloxybenzylique ou un allyl-triéthylène glycol ; éventuellement où R₂ représente, à chaque occurrence, un hydrogène ou un alkyle en C₁-C₁₀.

6. Composé polyaminoacide ayant une structure représentée par la Formule générale 5 : 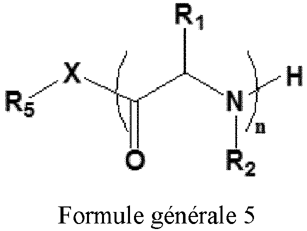 où
X représente un soufre ou un sélénium ;
R₁ représente indépendamment, à chaque occurrence, une chaîne latérale d'un acide aminé artificiel ;
R₂ représente indépendamment, à chaque occurrence, un hydrogène ou un alkyle en C₁-C₁₀, alcényle en C₂-C₁₀, alcynyle en C₂-C₁₀ ;
R₅ représente un alkyle en C₁-C₃₀ substitué ou non substitué, alcényle en C₂-C₃₀ substitué ou non substitué, alcynyle en C₂-C₃₀ substitué ou non substitué, cycloalkyle en C₃-C₃₀ substitué ou non substitué, cycloalcényle en C₃-C₃₀ substitué ou non substitué, hétéroalkyle en C₁-C₃₀ substitué ou non substitué, hétéroalcényle en C₂-C₃₀ substitué ou non substitué, hétéroalcynyle en C₂-C₃₀ substitué ou non substitué, hétérocycloalkyle en C₁-C₃₀ substitué ou non substitué, hétérocycloalcényle en C₂-C₃₀ substitué ou non substitué, aryle en C₆-C₃₀ substitué ou non substitué ou hétéroaryle en C₅-C₃₀ substitué ou non substitué ; et
n est un nombre entier sélectionné dans la plage de 1 à 200.

7. Composé polyaminoacide selon la revendication 6, où R₁ représente, à chaque occurrence, une chaîne latérale de la tyrosine, de la sérine, de la thréonine, de la cystéine, de l'acide aspartique et/ou de l'acide glutamique modifiée par un oligo(éthylène glycol), un phosphate, un ester propényloxybenzylique ou un allyl-triéthylène glycol ; éventuellement où R₂ représente, à chaque occurrence, un hydrogène ou un alkyle en C₁-C₁₀ ; éventuellement où R₅ représente un alkyle en C₁-C₁₀ substitué ou non substitué, alcényle en C₂-C₁₀ substitué ou non substitué, alcynyle en C₂-C₁₀ substitué ou non substitué, cycloalkyle en C₃-C₁₀ substitué ou non substitué, cycloalcényle en C₃-C₁₀ substitué ou non substitué, hétéroalkyle en C₁-C₁₀ substitué ou non substitué, hétéroalcényle en C₂-C₁₀ substitué ou non substitué, hétérocycloalkyle en C₁-C₁₀ substitué ou non substitué, hétérocycloalcényle en C₂-C₁₀ substitué ou non substitué, aryle en C₆-C₁₈ substitué ou non substitué ou hétéroaryle en C₅-C₁₈ substitué ou non substitué, où un substituant de R₅ est sélectionné dans le groupe consistant en un haloalkyle en C₁-C₃, hydroxy, amino, mercapto, carbonyle, carboxy, sulfo, carboxylate, sulfonate, groupement ester, amide et/ou halogène ; et préférablement où R₅ représente un alkyle en C₁-C₁₀ substitué ou non substitué, cycloalkyle en C₃-C₁₀ substitué ou non substitué, hétéroalkyle en C₁-C₁₀ substitué ou non substitué, hétérocycloalkyle en C₁-C₁₀ substitué ou non substitué, aryle C₆-C₁₈ en substitué ou non substitué ou hétéroaryle en C₅-C₁₈ substitué ou non substitué.

8. Composé polyaminoacide selon les revendications 6 ou 7, où R₅ est sélectionné parmi les structures suivantes :

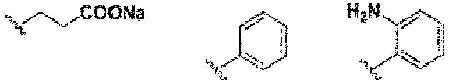

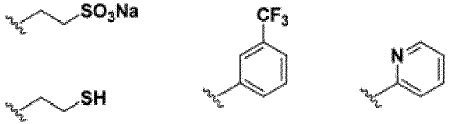

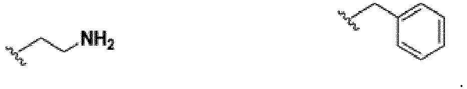

9. Composé polyaminoacide ayant une structure représentée par la Formule générale 6 : 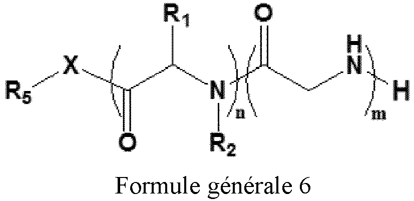 où
X représente un soufre ou un sélénium ;
R₁ représente indépendamment, à chaque occurrence, une chaîne latérale d'un acide aminé artificiel ;
R₂ représente indépendamment, à chaque occurrence, un hydrogène ou un alkyle en C₁-C₁₀, alcényle en C₂-C₁₀, alcynyle en C₂-C₁₀ ;
R₅ représente un alkyle en C₁-C₃₀ substitué ou non substitué, alcényle en C₂-C₃₀ substitué ou non substitué, alcynyle en C₂-C₃₀ substitué ou non substitué, cycloalkyle en C₃-C₃₀ substitué ou non substitué, cycloalcényle en C₃-C₃₀ substitué ou non substitué, hétéroalkyle en C₁-C₃₀ substitué ou non substitué, hétéroalcényle en C₂-C₃₀ substitué ou non substitué, hétéroalcynyle en C₂-C₃₀ substitué ou non substitué, hétérocycloalkyle en C₁-C₃₀ substitué ou non substitué, hétérocycloalcényle en C₂-C₃₀ substitué ou non substitué, aryle en C₆-C₃₀ substitué ou non substitué ou hétéroaryle en C₅-C₃₀ substitué ou non substitué ;
n est un nombre entier sélectionné dans la plage de 10 à 200 ; et
m est un nombre entier sélectionné dans la plage de 1 à 30.

10. Procédé de préparation d'un conjugué protéine-polyaminoacide selon l'une quelconque des revendications 1 à 3, comprenant : (1) l'initiation de la polymérisation d'un N-carboxyanhydride par un initiateur R₅XY pour obtenir un polyaminoacide ayant une structure carbonée ; et (2) le mélange du polyaminoacide et d'une protéine contenant une structure 1,2-mercaptoéthylamine pour obtenir le conjugué protéine-polyaminoacide spécifique d'un site par le biais d'une ligature chimique native,
où
X représente un soufre ou un sélénium ;
Y représente un hydrogène ou un trialkylsilyle, où le fragment alkyle du trialkylsilyle est un alkyle en Ci-Cio ; et
R₅ représente un alkyle en C₁-C₃₀ substitué ou non substitué, alcényle en C₂-C₃₀ substitué ou non substitué, alcynyle en C₂-C₃₀ substitué ou non substitué, cycloalkyle en C₃-C₃₀ substitué ou non substitué, cycloalcényle en C₃-C₃₀ substitué ou non substitué, hétéroalkyle en C₁-C₃₀ substitué ou non substitué, hétéroalcényle en C₂-C₃₀ substitué ou non substitué, hétéroalcynyle en C₂-C₃₀ substitué ou non substitué, hétérocycloalkyle en C₁-C₃₀ substitué ou non substitué, hétérocycloalcényle en C₂-C₃₀ substitué ou non substitué, aryle en C₆-C₃₀ substitué ou non substitué ou hétéroaryle en C₅-C₃₀ substitué ou non substitué.

11. Procédé selon la revendication 10 où, à l'étape (1), la polymérisation du N-carboxyanhydride est initiée par l'initiateur R₅XY pour obtenir un polyaminoacide PAA-XR₅ contenant une structure carbonée : 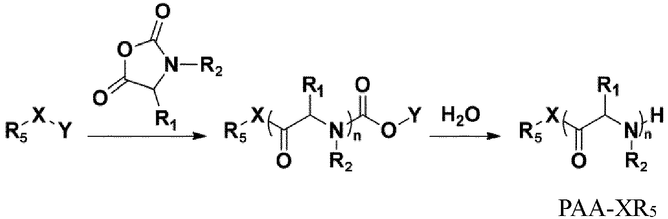 où
R₁ représente indépendamment, à chaque occurrence, une chaîne latérale d'un acide aminé artificiel ;
R₂ représente indépendamment, à chaque occurrence, un hydrogène ou un alkyle en C₁-C₁₀, alcényle en C₂-C₁₀, alcynyle en C₂-C₁₀ ; et
n est un nombre entier sélectionné dans la plage de 1 à 200.

12. Procédé selon la revendication 11, où R₂ représente un hydrogène.

13. Procédé selon la revendication 11 ou 12, où le R₅XY est le (triméthylsilyl)benzènethiol ou le (triméthylsilyl)benzènesélénol, éventuellement où l'étape (1) est effectuée dans un solvant aprotique à température ambiante, éventuellement où le polyaminoacide obtenu à l'étape (1) est soumis à une post-modification, la post-modification comprenant le mélange du polyaminoacide et d'un modificateur et l'exposition du mélange à une lampe ultraviolette et le modificateur étant le chlorhydrate de mercaptoéthylamine ou l'acide mercaptopropionique.

14. Procédé selon la revendication 12 où, à l'étape (2), le polyaminoacide obtenu à l'étape (1) est mélangé avec la protéine contenant une structure 1,2-mercaptoéthylamine pour obtenir le conjugué protéine-polyaminoacide spécifique d'un site par le biais d'une ligature chimique native : 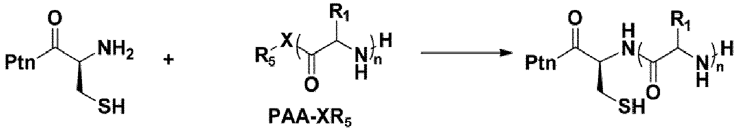 où Ptn représente une protéine.

15. Procédé selon la revendication 11 où, à l'étape (2), le polyaminoacide obtenu à l'étape (1) est mélangé avec la protéine contenant une structure 1,2-mercaptoéthylamine pour obtenir le conjugué protéine-polyaminoacide spécifique d'un site par le biais d'une ligature chimique native : 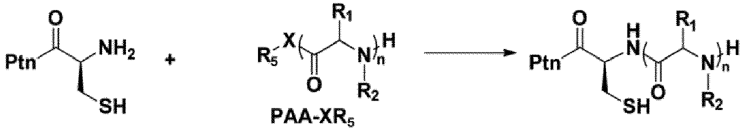 où Ptn représente une protéine.

16. Procédé de préparation d'un conjugué protéine-polyaminoacide cyclique selon la revendication 4 ou 5, comprenant les étapes suivantes : (1) initiation de la polymérisation d'un N-carboxyanhydride et d'un N-carboxyanhydride de glycine par un initiateur R₅XY pour obtenir un bloc polyaminoacide ayant une structure thioester de phényle à l'extrémité C-terminale et une structure à bloc polyglycine à l'extrémité N-terminale ; et (2) mélange du bloc polyaminoacide et d'une protéine ayant une cystéine à l'extrémité N-terminale et une séquence LPXaTG à l'extrémité C-terminale pour effectuer avec succès une ligature chimique native, et réaction sous la médiation d'une sortase pour produire la cyclisation de la protéine, où la séquence LPXaTG est un site de reconnaissance de la sortase,
où
X représente un soufre ;
Y représente un hydrogène ou un trialkylsilyle, où le fragment alkyle du trialkylsilyle est un alkyle en C₁-C₁₀ ; et
R₅ représente un groupement phényle.

17. Procédé selon la revendication 16 où, à l'étape (1), une polymérisation du N-carboxyanhydride et d'un autre N-carboxyanhydride est initiée par l'initiateur R₅XY pour obtenir un bloc polyaminoacide ayant une structure thioester de phényle à l'extrémité C-terminale et une structure à bloc polyglycine à l'extrémité N-terminale : 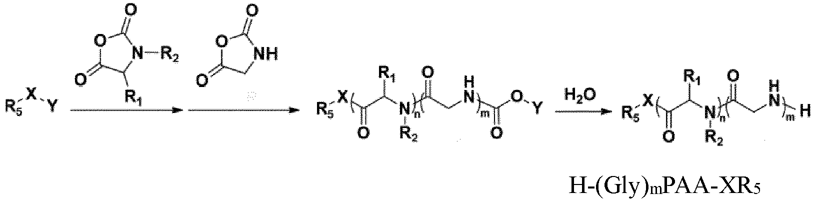 où
R₁ représente indépendamment, à chaque occurrence, une chaîne latérale d'un acide aminé artificiel ;
R₂ représente indépendamment, à chaque occurrence, un hydrogène ou un alkyle en C₁-C₁₀, alcényle en C₂-C₁₀, alcynyle en C₂-C₁₀ ;
n est un nombre entier sélectionné dans la plage de 10 à 200 ; et
m est un nombre entier sélectionné dans la plage de 1 à 30.

18. Procédé selon la revendication 17, où R₂ représente un hydrogène.

19. Procédé selon la revendication 17 ou 18, où le R₅XY est le benzènethiol ; éventuellement où l'étape (1) est effectuée dans un solvant aprotique à température ambiante ; éventuellement où le polyaminoacide obtenu à l'étape (1) est soumis à une post-modification, la post-modification comprenant le mélange du polyaminoacide et d'un modificateur et l'exposition du mélange à une lampe ultraviolette et le modificateur étant le chlorhydrate de mercaptoéthylamine ou l'acide mercaptopropionique.

20. Procédé selon la revendication 17 ou 18 où, à l'étape (2), le bloc polyaminoacide obtenu à l'étape (1) est mélangé avec la protéine ayant une cystéine à l'extrémité N-terminale et une séquence LPXaTG à l'extrémité C-terminale, le bloc polyaminoacide est ligaturé à la cystéine à l'extrémité N-terminale de la protéine puis une cyclisation est effectuée en la présence d'une sortase : 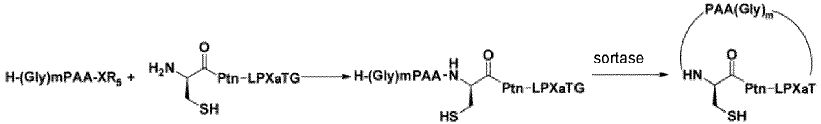 où la séquence LPXaTG est un site de reconnaissance de la sortase.


### SEQUENCE LISTING

<110> PEKING UNIVERSITY
<120> Poly(amino acid), Protein-Poly(amino acid) Conjugate and Preparation
   Method Thereof
<130> JWJ02477EP
<140> EP16881284.0
   <141>
<150> PCT/CN2016/113590
   <151> 2016-12-30
<150> 201511020841.X
   <151> 2015-12-30
<150> 201610523202.3
   <151> 2016-07-05
<160> 3
<170> PatentIn version 3.3
<210> 1
   <211> 275
   <212> PRT
   <213> Artificial Sequence
<220>
   <223> ENLYFQ-Cys-eGFP-LPETGH6
<400> 1
   Met Glu Asn Leu Tyr Phe Gln Cys Leu Asn Asp Ile Phe Glu Ala Gln
   1               5                   10                  15
   Lys Ile Glu Trp His Glu Met Val Ser Lys Gly Glu Glu Leu Phe Thr
               20                  25                  30
   Gly Val Val Pro Ile Leu Val Glu Leu Asp Gly Asp Val Asn Gly His
           35                  40                  45
   Lys Phe Ser Val Ser Gly Glu Gly Glu Gly Asp Ala Thr Tyr Gly Lys
       50                  55                  60
   Leu Thr Leu Lys Phe Ile Cys Thr Thr Gly Lys Leu Pro Val Pro Trp
   65                  70                  75                  80
   Pro Thr Leu Val Thr Thr Leu Thr Tyr Gly Val Gln Cys Phe Ser Arg
                   85                  90                  95
   Tyr Pro Asp His Met Lys Gln His Asp Phe Phe Lys Ser Ala Met Pro
               100                 105                 110
   Glu Gly Tyr Val Gln Glu Arg Thr Ile Phe Phe Lys Asp Asp Gly Asn
           115                 120                 125
   Tyr Lys Thr Arg Ala Glu Val Lys Phe Glu Gly Asp Thr Leu Val Asn
       130                 135                 140
   Arg Ile Glu Leu Lys Gly Ile Asp Phe Lys Glu Asp Gly Asn Ile Leu
   145                 150                 155                 160
   Gly His Lys Leu Glu Tyr Asn Tyr Asn Ser His Asn Val Tyr Ile Met
                   165                 170                 175
   Ala Asp Lys Gln Lys Asn Gly Ile Lys Val Asn Phe Lys Ile Arg His
               180                 185                 190
   Asn Ile Glu Asp Gly Ser Val Gln Leu Ala Asp His Tyr Gln Gln Asn
           195                 200                 205
   Thr Pro Ile Gly Asp Gly Pro Val Leu Leu Pro Asp Asn His Tyr Leu
       210                 215                 220
   Ser Thr Gln Ser Ala Leu Ser Lys Asp Pro Asn Glu Lys Arg Asp His
   225                 230                 235                 240
   Met Val Leu Leu Glu Phe Val Thr Ala Ala Gly Ile Thr Leu Gly Met
                   245                 250                 255
   Asp Glu Leu Tyr Lys Leu Pro Glu Thr Gly Gly Leu Glu His His His
               260                 265                 270
   His His His
           275
<210> 2
   <211> 246
   <212> PRT
   <213> Artificial Sequence
<220>
   <223> C-terminus His6-tagged amino acid sequence
<400> 2
   Met Gly Lys Gly Glu Glu Leu Phe Thr Gly Val Val Pro Ile Leu Val
   1               5                   10                  15
   Glu Leu Asp Gly Asp Val Asn Gly His Lys Phe Ser Val Ser Gly Glu
               20                  25                  30
   Gly Glu Gly Asp Ala Thr Tyr Gly Lys Leu Thr Leu Lys Phe Ile Cys
           35                  40                  45
   Thr Thr Gly Lys Leu Pro Val Pro Trp Pro Thr Leu Val Thr Thr Phe
       50                  55                  60
   Ser Tyr Gly Val Gln Cys Phe Ser Arg Tyr Pro Asp His Met Lys Arg
   65                  70                  75                  80
   His Asp Phe Phe Lys Ser Ala Met Pro Glu Gly Tyr Val Gln Glu Arg
                   85                  90                  95
   Thr Ile Ser Phe Lys Asp Asp Gly Asn Tyr Lys Thr Arg Ala Glu Val
               100                 105                 110
   Lys Phe Glu Gly Asp Thr Leu Val Asn Arg Ile Glu Leu Lys Gly Ile
           115                 120                 125
   Asp Phe Lys Glu Asp Gly Asn Ile Leu Gly His Lys Leu Glu Tyr Asn
       130                 135                 140
   Tyr Asn Ser His Asn Val Tyr Ile Thr Ala Asp Lys Gln Lys Asn Gly
   145                 150                 155                 160
   Ile Lys Ala Asn Phe Lys Ile Arg His Asn Ile Glu Asp Gly Ser Val
                   165                 170                 175
   Gln Leu Ala Asp His Tyr Gln Gln Asn Thr Pro Ile Gly Asp Gly Pro
               180                 185                 190
   Val Leu Leu Pro Asp Asn His Tyr Leu Ser Thr Gln Ser Ala Leu Ser
           195                 200                 205
   Lys Asp Pro Asn Glu Lys Arg Asp His Met Val Leu Leu Glu Phe Val
       210                 215                 220
   Thr Ala Ala Gly Ile Thr His Gly Met Asp Glu Leu Tyr Lys Gly Pro
   225                  230                    235                 240
   His His His His His His
                   245
<210> 3
   <211> 187
   <212> PRT
   <213> Artificial Sequence
<220>
   <223> TEV-Cys-IFN¦Á-LPETGLEH6
<400> 3
   Met Glu Asn Leu Tyr Phe Gln Cys Gly Cys Asp Leu Pro Gln Thr His
   1               5                   10                  15
   Ser Leu Gly Ser Arg Arg Thr Leu Met Leu Leu Ala Gln Met Arg Arg
               20                  25                  30
   Ile Ser Leu Phe Ser Cys Leu Lys Asp Arg His Asp Phe Gly Phe Pro
                       35                                      40                                      45
   Gln Glu Glu Phe Gly Asn Gln Phe Gln Lys Ala Glu Thr Ile Pro Val
           50                                      55                                      60
   Leu His Glu Met Ile Gln Gln Ile Phe Asn Leu Phe Ser Thr Lys Asp
   65                                      70                                      75                                      80
   Ser Ser Ala Ala Trp Asp Glu Thr Leu Leu Asp Lys Phe Tyr Thr Glu
                                   85                                      90                                      95
   Leu Tyr Gln Gln Leu Asn Asp Leu Glu Ala Cys Val Ile Gln Gly Val
                           100                                     105                                     110
   Gly Val Thr Glu Thr Pro Leu Met Lys Glu Asp Ser Ile Leu Ala Val
                   115                                     120                                     125
   Arg Lys Tyr Phe Gln Arg Ile Thr Leu Tyr Leu Lys Glu Lys Lys Tyr
           130                                     135                                     140
   Ser Pro Cys Ala Trp Glu Val Val Arg Ala Glu Ile Met Arg Ser Phe
   145                                     150                                     155                                     160
   Ser Leu Ser Thr Asn Leu Gln Glu Ser Leu Arg Ser Lys Glu Leu Pro
                                   165                                     170                                     175
   Glu Thr Gly Leu Glu His His His His His His
                           180                                     185